# EUROPÄISCHE PATENTSCHRIFT

(11) **EP 2 919 702 B1**
(45) Veröffentlichungstag und Bekanntmachung des Hinweises auf die Patenterteilung: **06.02.2019**
(21) Anmeldenummer: 13785354.5
(22) Anmeldetag: 18.10.2013
(51) Int. Cl.: A61C 8/00

(54) **BEHANDLUNGSELEMENT ZUR VERWENDUNG MIT EINEM DENTAL-IMPLANTAT-TEIL, BEHANDLUNGSSYSTEM UND VERFAHREN ZUR REINIGUNG EINES DENTAL-IMPLANTAT-TEILS**
TREATMENT ELEMENT FOR USE TOGETHER WITH A DENTAL IMPLANT PART, TREATMENT SYSTEM AND METHOD FOR CLEANING A DENTAL IMPLANT PART
ÉLÉMENT DE TRAITEMENT POUR UTILISATION AVEC UN COMPOSANT D'IMPLANT DENTAIRE, SYSTÈME DE TRAITEMENT ET PROCÉDÉ DE NETTOYAGE D'UN COMPOSANT D'IMPLANT DENTAIRE

(30) Priorität: 14.11.2012 DE 102012022227; 14.11.2012 DE 102012022593; 05.02.2013 DE 102013201883
(43) Veröffentlichungstag der Anmeldung: 23.09.2015
(73) Patentinhaber: Zyfoma GmbH, 64331 Weiterstadt (DE)
(72) Erfinder: ZIPPRICH, Holger, 64342 Malchen (DE)
(74) Vertreter: Harbach, Ulrich
(86) Internationale Anmeldenummer: PCT/EP2013/003150
(87) Internationale Veröffentlichungsnummer: WO 2014/075755

(56) Entgegenhaltungen:
- WO-A2-2005/102191
- US-A- 5 201 656
- US-A1- 2004 243 130
- US-A1- 2010 291 506
- US-A1- 2012 028 215
- US-A1- 2012 196 251

## Beschreibung

Die Erfindung betrifft ein Behandlungselement, insbesondere zur Verwendung mit einem in den Kieferknochen eines Patienten eingebrachten Dental-Implantat-Teil. Sie bezieht sich weiter auf ein Behandlungssystem mit einem derartigen Behandlungselement sowie auf ein Verfahren zur Reinigung eines Dental-Implantat-Teils.

Zum Ausgleich des Verlusts eines Zahnes können im Rahmen der rekonstruktiven Therapie Dentalimplantate zum Einsatz kommen. Sie werden üblicherweise anstelle eines extrahierten oder ausgefallenen Zahnes in den Kieferknochen eingesetzt, um dort nach einer Einheilphase von etwas vier bis zwölf Wochen ein als Zahnersatz dienendes prothetisches Teil oder eine Krone zu halten. Dazu ist ein derartiges Dentalimplantat üblicherweise als geeignet geformter Metallkörper ausgebildet, der durch Einschrauben an der vorgesehenen Stelle in den Kieferknochen eingesetzt wird. Das Dentalimplantat weist dabei in der Regel am apikalen Ende ein zumeist selbstschneidendes Schraubengewinde auf, mit welchem das Dentalimplantat in das entsprechend präparierte Implantatbett eingesetzt wird.

Angesichts der mittlerweile vergleichsweise hohen Anzahl der inserierten Implantate im menschlichen Körper, insbesondere im Dentalbereich, sowie deren verhältnismäßig langer Nutzungsdauer wurde ein stetiger Anstieg im Auftreten von biofilmassoziierten Entzündungszuständen des periimplantären Gewebes festgestellt. An der von Gewebe und Gewebeflüssigkeit umschlossenen festen Oberfläche des Implantats bildet sich ein Biofilm, der von Bakterien besiedelt wird, die letztlich zu chronischen und wiederkehrenden Infektionen führen können. Dieses Erkrankungsbild wird als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauhen Oberfläche des Pfostenteils und weiterer Faktoren ursächlich für das Vollbild der Periimplantitis, welche sich durch eine zunehmende Belastung und Zerstörung des Hart- und Weichgewebes auszeichnet. Die Bereiche, an denen sich das Hart- und/oder Weichgewebe zurückzieht, werden dabei in der Regel mit einem Biofilm überzogen.
Sowohl behandelt als auch unbehandelt kann ein Voranschreiten der periimplantären Entzündung zum Verlust des Implantates und zur Beeinträchtigung des Körper- oder Knochengewebes im Bereich der Einbringungsstelle führen. Es ist daher wünschenswert, möglichst frühzeitig nach Feststellung einer derartigen Entzündung geeignete Gegenmaßnahmen einzuleiten. Diese können von einer Optimierung der Mundhygiene bis hin zu therapeutischen Eingriffen in Form chirurgischer Maßnahmen, d. h. einer Entnahme des befallenen Implantats und Neueinsetzen eines Austauschimplantats, reichen. Insbesondere letztgenannte Maßnahme ist jedoch sehr belastend für das Gewebe insgesamt und geht oftmals mit massivem Gewebeabbau in der Umgebung der Insertionsstelle einher. Alternative wirksame Maßnahmen zur Bekämpfung existierender oder sich anbahnender Periimplantitis sind daher äußerst wünschenswert.

Aus US 2004/0243130 A1 ist eine Vorrichtung zur Elektro-Stimulation von Knochen bekannt.

Der Erfindung liegt daher die Aufgabe zugrunde, ein Behandlungselement zur Bekämpfung oder Eindämmung einer sich anbahnenden Periimplantitis anzugeben, das bereits im Vorfeld von eigentlichen Therapiemaßnahmen einsetzbar ist und diese möglicherweise ganz überflüssig macht. Des Weiteren sollen ein Behandlungssystem mit einem derartigen Behandlungselement und ein Verfahren angegeben werden, mit dem das Behandlungselement besonders günstig einsetzbar ist.
Bezüglich des Behandlungselements wird diese Aufgabe erfindungsgemäß gelöst mit den Merkmalen des Anspruchs 1.
Vorteilhafte Ausgestaltungen der Erfindung sind Gegenstand der Unteransprüche.

Die Erfindung geht von der Überlegung aus, dass für eine wirkungsvolle und effiziente Behandlung von Entzündungserscheinungen gerade im Umfeld eines inserierten Dentalimplantats therapeutische Maßnahmen dadurch vermieden oder zumindest vom Umfang her gering gehalten werden können, indem bereits frühzeitig konsequent die bakterielle Besiedelung des Implantats und seines Umfelds bekämpft wird. Dies sollte insbesondere bereits dann erfolgen, wenn noch keine therapeutischen Maßnahmen erforderlich sind. Eine derartige konsequente Bekämpfung des Bakterienbefalls sollte zwei Hauptansatzpunkte verfolgen, nämlich einerseits eine möglichst weit gehende und zuverlässige Abtötung von Bakterien und andererseits eine zuverlässige und umfassende Reinigung des Implantatmaterials, mit der möglicherweise anhaftende organische Reste oder dergleichen, die die Neuansiedlung von Bakterien begünstigen könnten, in weit reichendem Umfang entfernt werden.

Wie sich völlig überraschend herausgestellt hat, können diese beiden Ziele mit einem gemeinsamen Ansatz verfolgt werden, indem dem befallenen Raumbereich gezielt und lokalisiert eine geeignete Reinigungsflüssigkeit zugeführt wird, beispielsweise durch Nutzung einer geeignet gewählten bioziden Flüssigkeit oder dergleichen. Daher ist der Grundkörper des Behandlungselements mit einer Anzahl von Medienkanälen für eine Reinigungsflüssigkeit versehen.

Vorzugsweise sind diese dabei derart geführt und positioniert, dass eine zielgenaue Beaufschlagung des zu reinigenden Bereichs im inserierten Dental-Implantat-Teil ermöglicht ist. Dazu weist vorteilhafterweise der oder jeder Medienkanal eine Auslassöffnung auf, die bei auf das Dental-Implantat-Teil aufgesetztem Grundkörper in einem Abstand von nicht mehr als 10 mm, vorzugsweise nicht mehr als 5 mm, besonders bevorzugt nicht mehr als 3 mm, vom Dental-Implantat-Teil positioniert ist. In alternativer oder zusätzlicher vorteilhafter Weiterbildung ist der oder sind die Medienkanäle in der Art einer integrierten Bauweise direkt im oder am Grundkörper angeordnet.

Wie sich weiterhin überraschend herausgestellt hat, kann eine besonders hohe Reinigungswirkung am inserierten Implantat-Teil erreicht werden, indem mehrere verschiedene Reinigungsflüssigkeiten miteinander kombiniert oder sequentiell nacheinander verabreicht werden. Um dies gezielt und bedarfsgerecht zu ermöglichen, bilden die im oder am Grundkörper des Behandlungselements vorgesehenen Medienkanäle vorteilhafterweise ein Kanalsystem mit zumindest zwei voneinander unabhängig mit Flüssigkeit beaufschlagbaren Teilkanälen. In weiterer vorteilhafter Ausgestaltung ist zudem mindestens ein Rückströmkanal zur Rückführung oder Absaugung verbrauchter Reinigungsflüssigkeit aus dem Bereich der Insertionsstelle vorgesehen.

In besonders vorteilhafter Ergänzung ist zusätzlich vorgesehen, elektrischen Strom gezielt als Medium zum Abtöten der Bakterien und/oder zum Reinigen des befallenen Implantat-Teils zu nutzen. Insbesondere ist durch die Beaufschlagung des Implantat-Teils mit elektrischem Strom eine zuverlässige Entfernung möglicherweise anhaftenden organischen Materials erreichbar. Um dies unter Vermeidung therapeutischer Eingriffe, also insbesondere ohne chirurgische Entfernung des inserierten Implantats, zu ermöglichen, ist vorgesehen, den elektrischen Strom unmittelbar dem befallenen Implantat-Teil in noch inseriertem Zustand, d. h. ohne vorherige Entfernung aus dem Knochengewebe, zuzuführen.

Hierzu wird das bevorzugte Behandlungselement bereitgestellt, das auslegungsbedingt für eine Anbringung an das inserierte Dental-Implantat-Teil vorgesehen ist und demzufolge geeignet an das inserierte Dental-Implantat-Teil angepasste Verbindungsmittel aufweist. Das Behandlungselement ist dabei vorteilhafterweise dafür ausgelegt, den zu Reinigungszwecken des inserierten Implantat-Teils vorgesehen Stromfluss gezielt lokalisiert im unmittelbar benachbarten Raumbereich aufbringen zu können. Insbesondere ist dabei als Auslegungsprinzip für das Behandlungselement vorgesehen, Mittel zur Führung eines Stromflusses bereitzustellen, mit denen der elektrische Strom dem inserierten Implantat-Teil zugeführt und dessen inserierter Bereich, insbesondere der mit dem Gewinde versehene Einsetzbereich im Knochengewebe, als Elektrode verwendet werden kann. Zur Bildung eines Gegenpols oder der Gegenelektrode ist zudem in unmittelbarer Nachbarschaft die geeignete Positionierung einer Kontaktfläche oder eines elektrischen Kontaktpunkts vorgesehen. Die genannten Komponenten sollen dabei derart positioniert und ggf. geeignet mit einer Stromquelle verbunden werden, dass der zu Behandlungs- und Reinigungszwecken angelegte elektrische Strom durch die von den Bakterien befallene Oberflächenzone des inserierten Implantat-Teils hindurch- und von dort aus weitgehend unmittelbar, also insbesondere ohne "Umwege" über weiteres Körpergewebe oder dergleichen, zur Kontaktfläche bzw. zu dem Kontaktpunkt fließen kann.

Dazu umfasst das Behandlungselement vorteilhafterweise geeignet gewählte und positionierte Leitungselemente, die ihrerseits an eine geeignet gewählte Strom- oder Spannungsquelle anschließbar sind. Dabei ist auslegungsbedingt unter anderem vorgesehen, über eines der Leitungselemente eine elektrisch leitende Verbindung zum inserierten Implantat-Teil herzustellen, so dass der erwünschte Stromfluss durch das inserierte Implantat-Teil erfolgen kann und dieses die eine der Elektroden bilden kann.

Das in der Art einer Gegenelektrode vorgesehene, die andere Kontaktfläche für den Stromfluss bildende zweite Leiterelement soll dabei vorzugsweise räumlich in unmittelbarer Nähe des inserierten Implantat-Teils positioniert werden. Angestrebt ist dabei, dass der Stromfluss durch den Kontaktbereich des inserierten Implantat-Teils zum umgebenden Knochen- oder Weichgewebe und von dort möglichst unmittelbar zur Kontaktfläche erfolgt, ohne dass ein Stromfluss über zu große Bereiche des Körpergewebes notwendig wäre. Dazu ist das zweite Leiterelement vorzugsweise derart geometrisch gestaltet, dass eine weitgehend im Nahbereich der Insertionsstelle lokalisierte "elektrisch aktive" Zone entsteht, beispielsweise in Form einer nadel- oder drahtartigen Formgebung. In alternativer oder zusätzlicher vorteilhafter Weiterbildung ist das zweite Leiterelement, beispielsweise in Form eines Elektrodenkörpers, in der Art einer integrierten Ausführung im oder am Grundkörper des Behandlungselements angeordnet.

Die Leiterelemente können dabei in der Art "konventioneller" Elektroden als geeignet gewählte und geometrisch konfigurierte Elektroden, beispielsweise auf Metallbasis in Form metallischer Drähte oder dergleichen, ausgeführt sein. In diesem Fall ist die das zweite Leiterelement bildende Elektrode vorzugsweise mit einer elektrischen Isolierung ausgestattet, beispielsweise als kunststoffummantelter Draht, wobei zur Bildung des Kontakts oder der Kontaktfläche beispielsweise endseitig ein rein metallischer, nicht weitere isolierter Bereich vorgesehen sein kann. Alternativ ist es aber auch möglich, zumindest eines der Leiterelemente, vorzugsweise das zweite Leiterelement, auf der Basis der Nutzung elektrischer Leitfähigkeit von Flüssigkeiten, beispielsweise wässriger Lösung von Salzen oder dergleichen, bereitzustellen. In derartigen Fällen kann das jeweilige Leiterelement auch durch eine Anzahl von geeignet geführten Kanälen im oder am Behandlungselement gebildet sein, die im Einsatzfall mit einer geeignet gewählten elektrisch leitfähigen Flüssigkeit beaufschlagt werden, die ihrerseits dann wiederum geeignet, beispielsweise über eine geeignet positionierte Elektrode, elektrisch mit der Strom- oder Spannungsquelle verbunden wird. Der Kontakt oder die Kontaktfläche für den elektrischen Stromfluss wird in diesem Fall durch die endseitige Austrittsfläche des jeweiligen Kanals gebildet, über die die im Kanal geführte Flüssigkeit elektrisch mit der Umgebung des Kanals in Kontakt gelangen kann. Diese Austrittsfläche sollte somit geeignet im oben genannten Sinne in der näheren Umgebung des inserierten Implantat-Teils positioniert sein.

Dentalimplantate können als so genannte einteilige Implantate ausgeführt sein, die einen im Wesentlichen einstückigen Aufbau ihres Grundkörpers aufweisen. Um aber eine erleichterte Einbringung in den Patientenmund und insbesondere eine besonders weitgehende Vorbereitung der eigentlichen Prothese bei der Anbringung an das Implantat bereits im Vorfeld der Patientenbehandlung, beispielsweise in einem zahntechnischen Labor, zu ermöglichen, können Dentalimplantate auch mehrteilig ausgeführt sein. Insbesondere kann dabei ein grundsätzlich zweiteiliger Aufbau vorgesehen sein, wobei das Dental-Implantatsystem ein erstes, zur Einbringung in den Kieferknochen vorgesehenes, auch als eigentliches Implantat oder Pfostenteil bezeichnetes Implantat-Teil und zusätzlich zu diesem ein zugeordnetes zweites, auch als Aufbauteil oder Abutment bezeichnetes zweites Implantat-Teil umfasst, an das wiederum das als Prothese oder dergleichen vorgesehene Zahnersatzstück anbringbar ist. Das erste Implantat-Teil oder Pfostenteil ist an seiner Außenseite üblicherweise mit einem Gewinde versehen, welches als selbstschneidendes oder auch als nicht selbstschneidendes Gewinde ausgeführt sein kann. Üblicherweise ist dabei der Bereich, welcher in den Knochen oder das Knochengewebe einwachsen soll, oberflächlich aufgeraut oder beschichtet.

Das Aufbauteil oder Abutment ist dabei üblicherweise über eine geeignet gewählte Verbindungsschraube mit dem Pfostenteil verschraubt. Bei der Einbringung wird dabei das Gewinde der Verbindungsschraube in ein zugeordnetes Innengewinde im Pfostenteil eingeschraubt. Der Schraubenkopf der Verbindungsschraube presst dabei beim Einschrauben über eine Stirnsenkung des Aufbauteils dieses auf das Pfostenteil. In besonders vorteilhafter Ausgestaltung ist das Behandlungselement zum Einsatz für ein derartiges zwei- oder mehrteiliges Dentalimplantat vorgesehen. Dazu umfasst sein Verbindungssystem zweckmäßigerweise eine Verbindungsschraube zur Einbringung in den Schraubenkanal des Pfostenteils eines zwei- oder mehrteilig ausgeführten Dental-Implantatsystems. In dieser Ausführungsform kann das Behandlungselement somit als "Behandlungsabutment" bezeichnet werden, das anstelle des eigentlich vorgesehenen Abutments eines mehrteiligen Dental-Implantatsystems auf dessen Pfostenteil aufgesetzt und mit diesem verbunden wird.

Um bei einem derartigen Aufbau einen elektrisch besonders zuverlässigen und wirksamen Zugang oder Kontakt zum Insertionsbereich des inserierten Pfostenteils, also insbesondere dessen metallischen Grundkörper, herzustellen, ist das Behandlungselement in weiterer vorteilhafter Ausgestaltung derart ausgeführt, dass die Verbindungsschraube elektrisch leitend mit dem ersten Leiterelement verbunden ist.

Vorteilhafterweise ist der Grundkörper des Behandlungselements an seiner Kontaktfläche zum Dental-Implantat-Teil elektrisch isoliert ausgeführt. Damit ist sichergestellt, dass die Strom- oder Potenzialführung in der gewünschten Weise, nämlich mit der gezielten Einbindung des inserierten Bereichs des Dental-Implantat-Teils in die Stromführung, erfolgen kann. Der Grundkörper kann dabei an sich auf der Basis eines elektrisch isolierenden Materials, beispielsweise als Keramikkörper oder Kunststoffkörper, ausgeführt sein, wobei in diesem Fall die Elektroden durch geeignet positionierte Metall-Komponenten oder geeignet geführte Flüssigkeitskanäle ausgeführt sein können. Alternativ kann der Grundkörper auch als Metall-Körper, beispielsweise aus Titan, ausgeführt sein. In diesem Fall kann die Isolationswirkung zum Dental-Implantat-Teil durch Anbringung eines geeigneten Isolatorelements, insbesondere einer eigenständigen Komponente oder auch einer Oberflächenbeschichtung, erreicht werden.

Um die angestrebte Strom- oder Potenzialführung in unmittelbarer räumlicher Nähe zur Insertionsstelle des Implantat-Teils besonders zu begünstigen, ist in vorteilhafter Weiterbildung das zweite Leiterelement des Behandlungselements in einer Längsrichtung im Wesentlichen parallel zur Zentralachse des Grundkörpers verschiebbar an diesem gelagert. Bei einer Ausgestaltung als Elektrode kann diese dabei insbesondere als nadelartiges Element, beispielsweise in Form eines dünnen Drahtes oder dergleichen, ausgeführt sein, der nach der Montage des Behandlungselements am Dental-Implantat-Teil zum Insertionsbereich hin vorgeschoben werden kann. Bei einer Ausgestaltung als eine leitfähige Flüssigkeit führendem Kanal kann stattdessen beispielsweise ein dünnes Rohr, eine Kanüle oder dergleichen vorgesehen sein, das bzw. die ebenfalls nach der Montage des Behandlungselements am Dental-Implantat-Teil derart zum Insertionsbereich hin vorgeschoben werden kann, dass seine/ihre Austrittsfläche hinreichend nah am behandlungsbedürftigen Raumbereich des inserierten Implantat-Teils positioniert wird.

In alternativer oder zusätzlicher vorteilhafter Weiterbildung ist das zweite Leiterelement derart am Grundkörper angebracht, dass sein endseitig angeordneter Kontakt in seitlicher Richtung gesehen in einem Abstand von höchstens 10 mm, vorzugsweise von höchstens 5 mm, von der zentralen Längsachse des Dental-Implantat-Teils und in zusätzlicher oder weiterer vorteilhafter Ausgestaltung in seitlicher Richtung gesehen in einem Abstand von mindestens 1 mm, vorzugsweise mindestens 1,5 mm, von der zentralen Längsachse des Dental-Implantat-Teils positionierbar ist. Damit ist die angestrebte Erzeugung vergleichsweise hoher elektrischer Stromdichten unmittelbar an der Insertionsstelle des Implantat-Teils bei ansonsten gering gehaltener Strombelastung des weiteren Körpergewebes besonders günstig erreichbar.

Wie bereits ausgeführt, können die Leiterelemente in der Art "konventioneller" Elektroden als geeignet gewählte und geometrisch konfigurierte Elektroden, beispielsweise auf Metallbasis, ausgeführt sein. Die alternative Ausgestaltung auf der Basis der Nutzung elektrischer Leitfähigkeit von Flüssigkeiten wird aber als besonders vorteilhaft angesehen, da damit einerseits eine vergleichsweise hohe Flexibilität bei der Kanalführung und damit auch bei der Strom- oder Potenzialführung erreichbar ist.

Bezüglich des Behandlungssystems für ein Dental-Implantat-Teil wird die genannte Aufgabe gelöst mit einem Behandlungselement der vorgenannten Art, dessen Leiterelemente elektrisch mit einer Strom- oder Spannungsquelle verbunden sind.

Wie sich völlig überraschend herausgestellt hat, ist dabei eine Beaufschlagung des von Bakterien befallenen Implantats mit Strom- oder Spannungspulsen besonders hochgradig wirksam, insbesondere was die Entfernung organischer Reste, die nach Abtötung der Bakterien noch an dem Material anhaften, betrifft. Daher ist die Strom- oder Spannungsquelle des Behandlungssystems in besonders vorteilhafter Ausgestaltung für eine bedarfsweise pulsierende Beaufschlagung der Leiterelemente mit Strom oder Spannung ausgelegt. Besonders bevorzugt ist dabei eine Arbeitsspannung an den Elektroden von bis zu 30 V vorgesehen.

Um die als besonders vorteilhaft angesehene Kombination einer elektrischen Behandlung des inserierten Dental-Implantat-Teils mit einer Flüssigkeits-basierten Behandlung zu ermöglichen, ist an das Behandlungselement des Behandlungssystems vorteilhafterweise ein Zuleitungssystem für eine Reinigungsflüssigkeit oder eine Kombination mehrerer Reinigungsflüssigkeiten angeschlossen. Besonders bevorzugt ist dabei als Reinigungsflüssigkeit Wasser, versetzt mit mindestens einer Säure und/oder mindestens einem Salz, vorgesehen. Besonders bevorzugt ist dabei als Säure Phosphorsäure, Zitronensäure, Ameisensäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium-, Zinn- oder Kaliumiodid, -chlorid, -nitrat, -carbonat oder -hydrogencarbonat und/oder Ammoniumchlorit, -nitrat oder -iodid oder eine Kombination von diesen vorgesehen.

Bezüglich des Verfahrens zur Reinigung eines Dental-Implantat-Teils wird die genannte Aufgabe gelöst, indem an das Dental-Implantat-Teil eine elektrische Spannung angelegt wird, und indem es mit einer Reinigungsflüssigkeit gespült wird. Vorteilhafterweise wird die elektrische Spannung dabei gepulst angelegt. In alternativer oder zusätzlicher vorteilhafter Weiterbildung wird dabei als Reinigungsflüssigkeit der oben genannten Art verwendet.

Die mit der Erfindung erzielten Vorteile bestehen insbesondere darin, dass durch die Ausgestaltung des Behandlungselements mit den Leiterelementen, insbesondere und besonders bevorzugt in Kombination mit geeignet geführten Kanälen zum Zuführen einer Reinigungsflüssigkeit, eine gezielte und lokalisierte Beaufschlagung des Dental-Implantat-Teils mit geeigneten Strom- oder Spannungspulsen erfolgen kann, ohne dass hierzu eine Entnahme aus dem Patientenmund oder ein anderweitiger therapeutischer Eingriff notwendig wäre. Damit ist in der Art einer vorbeugenden oder eindämmenden Maßnahme eine frühzeitige Bekämpfung von Bakterienansiedelungen auf dem inserierten Implantat-Teil möglich, bei der die überraschend aufgefundene Wirksamkeit elektrischer Ströme bei der Abtötung von Bakterien, aber auch bei der Abführung des am inserierten Implantatkörper möglicherweise noch anhaftenden organischen Materials, nutzbar gemacht wird.

Ein Ausführungsbeispiel der Erfindung wird anhand einer Zeichnung näher erläutert. Darin zeigen:
- FIG. 1, 2: jeweils ein Dental-Implantatsystem,
- FIG. 3, 4: jeweils ein Implantat-Teil oder Pfostenteil des Dental-Implantatsystems,
- FIG. 5: ein Behandlungselement für ein in den Kieferknochen eines Patienten eingebrachtes Dental-Implantat-Teil in perspektivischer Ansicht,
- FIG. 6, 7: jeweils eine Variante des Behandlungselements nach FIG. 5 im Längsschnitt,
- FIG. 8, 9: jeweils eine weitere Variante eines Behandlungselements in perspektivischer Ansicht,
- FIG. 10: das Behandlungselement nach FIG. 9 im Längsschnitt, und
- FIG. 11: systematisch ein zur Verwendung des Behandlungselements ausgelegtes Behandlungssystem.

Gleiche Teile sind in allen Figuren mit denselben Bezugszeichen versehen.

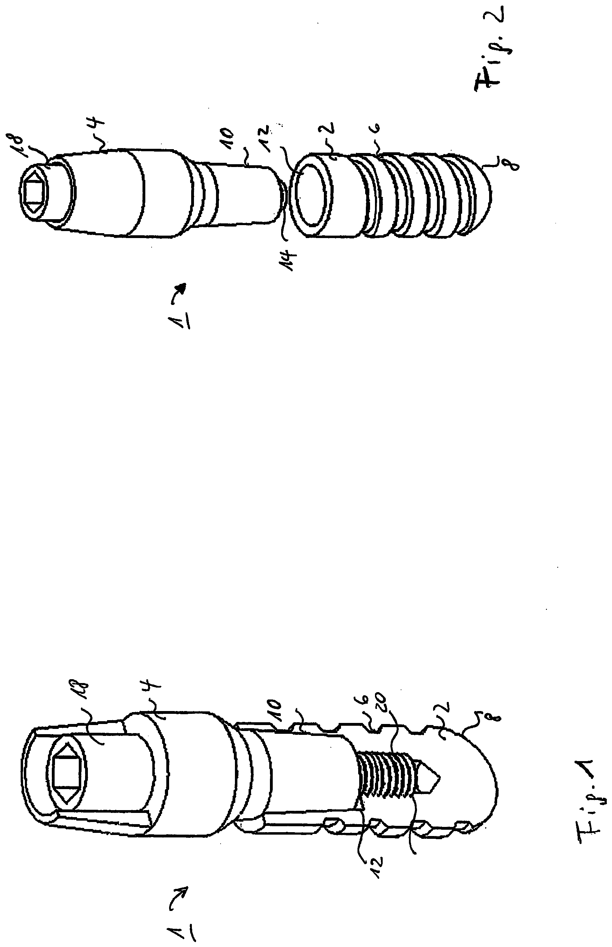

Das Dental-Implantatsystem 1 gemäß FIG. 1 ist zum Einsatz in den Kieferknochen anstelle eines extrahierten oder ausgefallenen Zahnes vorgesehen, um dort ein als Zahnersatz dienendes prothetisches Teil oder eine Krone zu halten. Das Dental-Implantatsystem 1 ist dabei mehrteilig ausgeführt und umfasst ein als so genanntes Pfostenteil ausgeführtes erstes Implantat-Teil 2 und ein diesem zugeordnetes, zur Anbringung eines Zahnersatzstücks vorgesehenes, auch als Aufbauteil oder Abutment bezeichnetes zweites Implantat-Teil 4. Das erste Implantat-Teil 2 oder Pfostenteil ist dabei außenseitig mit einem Außengewinde 6 versehen, das insbesondere am apikalen Ende 8 als selbstschneidendes Schraubengewinde ausgestaltet ist. Damit kann das erste Implantat-Teil 2 oder Pfostenteil durch Einschrauben an der vorgesehenen Stelle in den Kieferknochen eingesetzt werden.

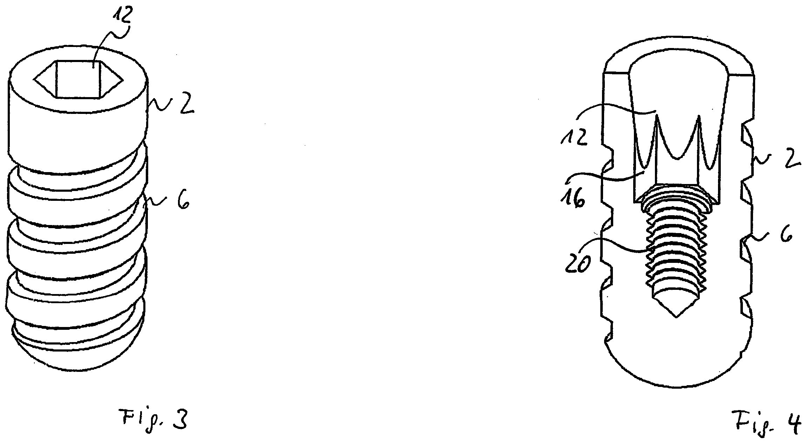

Um nach geeigneter Anbringung des Zahnersatzstücks oder der Prothese am Aufbauteil oder zweiten Implantat-Teil 4 ein Einbringen in das Pfostenteil oder erste Implantat-Teil 2 bei hoher mechanischer Stabilität zu ermöglichen, ist an das zweite Implantat-Teil 4 ein Verbindungszapfen 10 angeformt, der in einen zugeordneten, im ersten Implantat-Teil 2 vorgesehenen Aufnahmekanal 12 einschiebbar ist. Durch das Einschieben des Verbindungszapfens 10 in den Aufnahmekanal 12 entsteht eine mechanische Verbindung der Implantat-Teile 2, 4 miteinander. Für eine hohe mechanische Stabilität ist dabei der Verbindungszapfen 10 in seiner Außenkontur an die Innenkontur des Aufnahmekanals 12 angepasst, wobei beide in Längsrichtung gesehen konisch geformt sein können (Ausführungsbeispiel gemäß FIG. 2). Zudem kann, wie dies insbesondere im Ausführungsbeispiel gemäß FIG. 3 vorgesehen ist, die Außenkontur des Verbindungszapfens 10 - und dementsprechend angepasst die Innenkontur des Aufnahmekanals 12 - im Querschnitt mit einer mehrzähligen (im Ausführungsbeispiel sechszähligen) Symmetrie ausgestaltet sein, so dass beim Zusammenfügen der genannten Komponenten ein rotatorisches Gesperre entsteht und damit eine zuverlässige rotatorische Ausrichtung des Aufbauteils relativ zum Pfostenteil eingestellt werden kann. Im Ausführungsbeispiel gemäß FIG. 3, 4 ist zu diesem Zweck einer Indizierung oder zur Ausbildung eines rotatorischen Gesperres endseitig am Verbindungszapfen 10 ein Indizierungselement 14 mit seinerseits einem Querschnitt mit mehrzähliger Symmetrie angeordnet, das im montierten Zustand in ein entsprechendes, zugeordnetes Endkanalstück 16 im Aufnahmekanal 12 eingreift.

Das Dental-Implantatsystem 1 ist im Ausführungsbeispiel für eine Schraubverbindung der Implantat-Teile 2, 4 miteinander ausgeführt. Dazu ist jeweils eine Verbindungsschraube 18 vorgesehen, die in ein innerhalb des ersten Implantat-Teils 2 jeweils vorgesehenes Schraubgewinde 20 eingreift. Hinsichtlich ihrer Materialwahl sind die Implantat-Teile 2, 4 geeignet an den Einsatzzweck angepasst und grundsätzlich aus keramischem Material wie beispielsweise Zirkonoxid oder Aluminiumoxid oder auch aus einem geeignet gewählten Metall wie beispielsweise Titan gefertigt.

Generell besteht bei Dental-Implantatsystemen, insbesondere auch bei zweiteiligen Implantatsystemen der vorstehend beschriebenen Art, das Problem, dass durch ein Eindringen von Bakterien oder Keimen in den Gewebebereich in der Nähe der Insertionsstelle, insbesondere im Bereich des in den Kiefer eingebrachten Außengewindes 6, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem, also insbesondere auch das bereits inserierte Pfostenteil oder zweite Implantat-Teil 4, wieder aus dem Knochen entfernt und durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall soweit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere des Pfostenteils 2 besiedeln, haften aber in der Regel bevorzugt direkt an der Oberfläche des in den Kieferknochen inserierten Pfostenteils 2 im Kontaktbereich mit dem umgebenden Gewebe oder Knochenmaterial an, also insbesondere im Bereich des Außengewindes 6. In dessen Bereich kann die Oberfläche des Pfostenteils 2 mit einer Aufrauhung oder dergleichen versehen sein, um das Einwachsen in das Gewebe oder den Knochen besonders zu begünstigen und das Einheilen des Pfostenteils 2 nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis unter Erhaltung des bereits eingesetzten Implantatsystems, also insbesondere des bereits inserierten Pfostenteils 2, wirksam den Entzündungsherd bekämpfen und die eingedrungenen Keime abtöten zu können, so dass sich anschließend wieder gesundes Gewebe oder gesunde Knochensubstanz im Bereich um das Außengewinde 6 herum bilden kann. Dazu ist wünschenswert, zusätzlich zu einem gezielten Abtöten der Keime oder Bakterien im betroffenen Bereich auch noch deren Materialreste und Fragmente zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Pfostenteils 2 und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

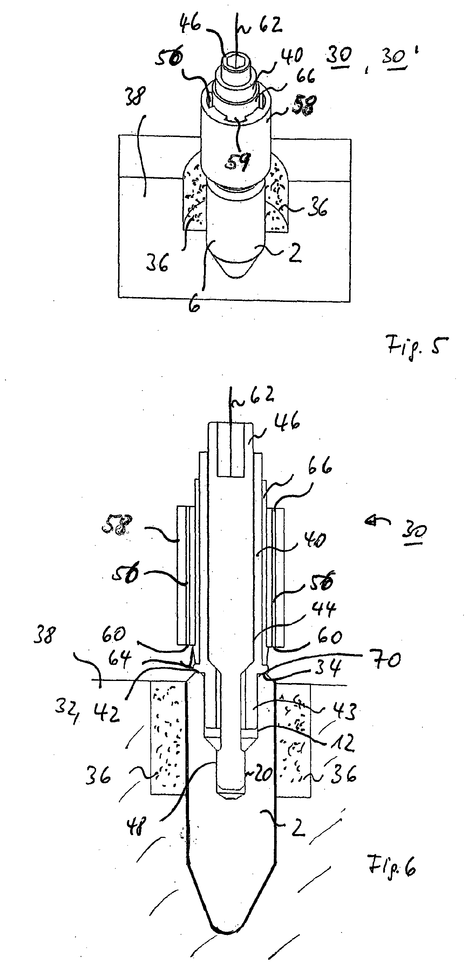

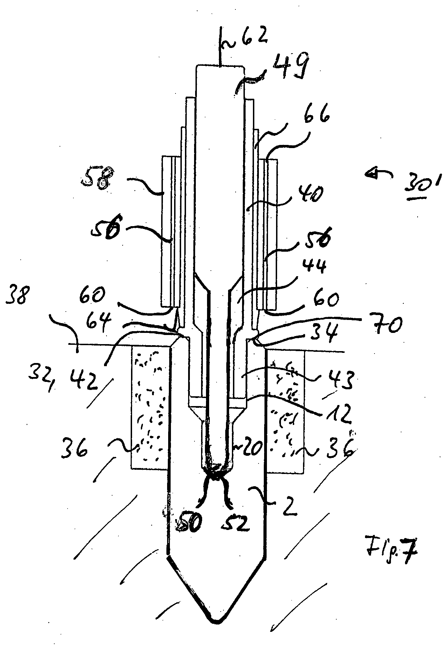

Zu diesem Zweck, also zum Abtöten von Keimen oder Bakterien im Insertionsbereich des Pfostenteils 2 und insbesondere auch zum anschließenden Ausspülen, Entfernen und Austragen der Gewebe- und Materialreste der abgetöteten Bakterien, ist ein Behandlungselement 30, 30' vorgesehen, wie es in perspektivischer Ansicht in FIG. 5 gezeigt ist. Eine erste bevorzugte Variante des Behandlungssystems 30 ist dabei im Längsschnitt in FIG. 6 und eine zweite bevorzugte Variante des Behandlungssystems 30' ist im Längsschnitt in FIG. 7 gezeigt.

Das Behandlungselement 30, 30' ist dabei im Ausführungsbeispiel aufgrund der an sich zweiteiligen Ausführung des Implantatsystems 1 in der Art eines Behandlungsabutments ausgeführt und zur Durchführung der genannten Behandlung für das dargestellte zweiteilig ausgeführte Implantatsystem 1 vorgesehen, wobei ein vorübergehendes Aufsetzen des Behandlungsabutments 30,30' auf das Pfostenteil 2 anstelle des eigentlichen Abutments oder zweiten Implantat-Teils 4 erfolgen soll. Die nachfolgenden Ausführungen beziehen sich daher auf diesen Fall eines zweiteiligen Implantatsystems 1; selbstverständlich kann aber auch in analoger Ausgestaltung eine entsprechende Verwendung für einteilige Implantate vorgesehen sein; hierfür müsste lediglich die mechanische Verbindung des Behandlungselements 30, 30' mit dem auch während der Behandlung im Kieferknochen verbleibenden Teil des Implantatsystems geeignet ausgestaltet sein, beispielsweise über eine geeignete Kontaktfläche, mit der das Behandlungselement 30, 30' anstelle der Prothetik auf das Abutment des Implantats aufgesetzt werden kann. Ebenso kann alternativ das Behandlungselement 30, 30' oben auf das eigentliche Abutment 4 des Implantatsystems 1 aufgesetzt werden, so dass eine Verwendung beispielsweise zur Bekämpfung einer Entzündung des Weichgewebes (Mukositis) durch Abtötung der Bakterien und Reinigung der Oberfläche vorgesehen sein kann, ohne dass dazu das eigentliche Abutment 4 entfernt werden müsste.

Bei der im Ausführungsbeispiel vorgesehenen zweiteiligen Ausführung des Implantatsystems 1 wird zur Durchführung der nachfolgend näher beschriebenen Behandlung zunächst - ggf. nach Entfernen der am eigentlichen Abutment oder zweiten Implantat-Teil 4 angebrachten Prothetik - die Schraubverbindung zwischen erstem und zweitem Implantat-Teil 2, 4 gelöst und das zweite Implantat-Teil 4 abgenommen. Das erste Implantat-Teil oder Pfostenteil 2 verbleibt dabei im Kieferknochen. Anschließend wird das Behandlungsabutment 30, 30' anstelle des eigentlichen Abutments 4 auf das Pfostenteil 2 aufgesetzt.

Die in den FIG. 6 und 7 dargestellten Varianten des Behandlungselements 30,30' sind im Wesentlichen baugleich, unterscheiden sich voneinander aber in der Art und Weise, wie während der eigentlichen Behandlung die Befestigung am Pfostenteil 2 vorgenommen werden soll. In der Variante gem. FIG. 6 ist eine Befestigung unter Nutzung der Schraubverbindung vorgesehen, wohingegen in der Variante gem. FIG. 7 lediglich ein Aufstecken auf das Pfostenteil 2 vorgesehen ist.

Das in FIG. 6 dargestellte Behandlungselement 30 wird somit nach dem Aufsetzen auf das Pfostenteil 2 über die Schraubverbindung mit diesem verbunden, wohingegen das in FIG. 7 dargestellte Behandlungselement 30' beim Aufsetzen auf das Pfostenteil 2 in dieses eingesteckt wird. Das Behandlungselement 30, 30' weist dazu in beiden Varianten eine im Wesentlichen plane Kontaktfläche 32 auf, mit der es auf die Stirnkante 34 des Pfostenteils 2 aufgesetzt werden kann. Die Kontaktfläche 32 kann unter Umständen auch die Funktion einer Dichtfläche erfüllen und dementsprechend ausgestaltet sein; insbesondere kann sie hierzu konisch ausgeführt sein.

Das Behandlungsabutment 30, 30' beruht hinsichtlich seiner Ausgestaltung und prinzipiellen Ausführung auf zwei jeweils als eigenständig erfinderisch angesehenen Grundkonzepten: einerseits ist es dafür ausgestaltet, die im Insertionsbereich des Pfostenteils 2 vorhandenen Keime oder Bakterien durch gezielte Zuführung eines bakterioziden, aber für den menschlichen Organismus verträglichen Reinigungs- oder Desinfektionsmittels gezielt abzutöten. Andererseits ist es dafür ausgelegt, eventuell an der Oberfläche des Pfostenteils, insbesondere im Bereich Außengewindes 6, noch anhaftende Reste oder Fragmente von Keimen und/oder Bakterien durch gepulste Beaufschlagung mit Stromstößen von der Außenoberfläche des Pfostenteils 2 abzulösen, so dass sie anschließend ausgewaschen werden können.

In einem ersten, sowohl bezüglich der Ausgestaltung des Systems als auch bezüglich der vorgesehenen Verfahrensschritte des Behandlungsverfahrens eigenständig als erfinderisch angesehenen Aspekt ist das Behandlungselement 30, 30' daher sowohl strukturell als auch funktional/konzeptionell dafür ausgelegt, eine Behandlungsflüssigkeit zum Abtöten der Keime oder Bakterien und/oder zum Reinigen des inserierten Implantat-Teils 2 gezielt in den Insertionsbereich des Pfostenteils 2, insbesondere den Bereich von dessen Außengewinde 6, einzuspeisen. Als Behandlungsflüssigkeit ist dabei besonders bevorzugt Wasser, versetzt mit mindestens einer Säure und/oder mindestens einem Salz, vorgesehen. Besonders bevorzugt ist dabei als Säure Phosphorsäure, Zitronensäure, Ameisensäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium-, Zinn- oder Kaliumiodid, -chlorid, -nitrat, -carbonat oder -hydrogencarbonat und/oder Ammoniumchlorit, -nitrat oder -iodid oder eine Kombination von diesen vorgesehen. Die Einspeisung der Reinigungsflüssigkeit soll dabei nahe zum Behandlungselement 30, 30' und damit in unmittelbarer Nachbarschaft zum betroffenen Raumbereich, also dem Insertionsbereich des Pfostenteils 2, erfolgen.

In einem zweiten, ebenfalls sowohl bezüglich der Ausgestaltung des Systems als auch bezüglich der vorgesehenen Verfahrensschritte des Behandlungsverfahrens eigenständig erfinderischen Aspekt ist das Behandlungselement 30, 30' dafür ausgelegt, die abgetöteten Bakterien oder Keime bzw. deren Reste oder Fragmente zuverlässig von der Außenoberfläche des Pfostenteils 2 abzulösen, so dass sie anschließend ausgespült werden können und sich nachfolgend gesundes Gewebe oder Knochenmaterial an die Oberfläche des Pfostenteils 2 anlegen und dieses wieder vollständig in gesundes Gewebe oder Knochenmaterial einwachsen kann. Für die Ablösung der Bakterien oder Keime bzw. deren Resten oder Fragmenten von der Oberfläche ist deren Benetzung mit einer leitfähigen Flüssigkeit unter Beaufschlagung mit gepulsten Stromstößen vorgesehen. Wie sich nämlich ebenfalls völlig überraschend herausgestellt hat, scheint gerade diese gepulste Beaufschlagung mit Stromstößen besonders zuverlässig das Ablösen der Bakterien oder Keime bzw. von deren Fragmenten oder Resten von der darunterliegenden Oberfläche zu bewirken, selbst wenn diese aufgeraut ist und eigentlich das Anhaften organischen Materials aufgrund ihrer Oberflächenstruktur besonders begünstigt.

Dabei liegt die überraschende Erkenntnis zugrunde, dass die Beaufschlagung des Pfostenteils 2 selbst mit gepulsten Stromstößen im Bereich von dessen Außenoberfläche, also insbesondere im Bereich des Außengewindes 6, zu einem Herauslösen von Materialfragmenten des Pfostenmaterials selbst, also beispielsweise Titan, aus der Oberfläche führt. Insbesondere führt dabei die Beaufschlagung mit den Stromstößen, gerade bei geeignet gewählter leitfähiger Flüssigkeit, beispielsweise mit Ionen- oder Säurebestandteilen, zu einer Bildung von TiO₂-Bereichen, die sich anschließend aus der Ti-Oberfläche herauslösen lassen. Durch diese Herauslösung von Oberflächenbestandteilen aus dem Pfostenteil 2 werden die oberflächlich anhaftenden Bestandteile oder Fragmente der Keime oder Bakterien mit abgelöst und restlos entfernt, so dass sie keine Basis und keinen Nährboden für eine Neuansiedlung von Keimen in diesen Bereichen bilden können. Zurück bleibt eine von Keimen, Bakterien oder deren Bestandteilen oder Resten befreite, angeraute und poröse Oberfläche des Pfostenteils 2, die gut als Basis für eine zukünftige Integration in das nachwachsende Knochengewebe dienen kann. Die verbleibende Oberfläche kann dabei auch durch eine Titan-Oxidschicht gebildet sein, die auch bei einem Anodisieren der Oberfläche entstehen würde.

Eine weitere Begünstigung dieser im Sinne einer zuverlässigen Reinigung der Oberfläche erwünschten Ablösung der Oberflächenbestandteile aus dem inserierten Pfostenteil 2 ist durch eine vorteilhafte besonders geeignete Verfahrensführung bei der Beaufschlagung mit Strom erreichbar. Diese kann derart vorgenommen werden, dass infolge des Stromflusses eine elektrolytische Gasblasenbildung im Bereich der inserierten Oberfläche stattfindet. Hierbei kann das Pfostenteil 2 anodisch oder auch kathodisch geschaltet werden. Insbesondere bei einer zumindest vorübergehend kathodischen Beschaltung des Pfostenteils 2 entstehen elektrolytisch induziert die Gase Wasserstoff, Sauerstoff, Stickstoff und/oder Kohlendioxid. Die dadurch gebildeten Gasblasen steigen in der umgebenden Flüssigkeit auf und erzeugen dadurch Mitnahmeeffekte, über die die genannten Oberflächenbestandteile mit abgetragen und nach außen hin abgefördert werden. Beispielsweise wurde völlig überraschend beobachtet, dass bei Verwendung einer positive Ionen enthaltender Lösung, beispielsweise einer wässrigen Salzlösung, diese Ionen sich bei einer kathodischen Beschaltung des Pfostenteils 2 an diesem abscheiden und damit die Gasblasenbildung deutlich verstärken. Beispielsweise führt die Anwesenheit von Na+-Ionen bei kathodischer Verschaltung des Pfostenteils 2 zu erheblicher Gasblasenbildung, da Na sofort zur Oxidation führt.

In einem dritten, ebenfalls sowohl bezüglich der Ausgestaltung des Systems als auch bezüglich der vorgesehenen Verfahrensschritte des Behandlungsverfahrens eigenständig erfinderischen Aspekt ist das Behandlungselement 30, 30' für eine besonders einfach und effizient gehaltene Kombination der genannten Aspekte ausgestaltet. Dabei liegt das Konzept zugrunde, dass sowohl die vorgesehene Zuführung der Reinigungsflüssigkeit als auch das gezielte Ablösen der Bakterien- und Keimreste und -fragmente durch Aufbringung der genannten Strompulse in einem gemeinsamen System und somit mit besonders einfach gehaltenen Mitteln erreicht werden kann.

Im Hinblick auf diese Auslegungsziele weist das Behandlungselement 30, 30' einen konstruktiven Aufbau auf, wie er den Darstellungen in perspektivischer Ansicht gemäß FIG. 5 und im Längsschnitt gemäß FIG. 6 bzw. FIG. 7 entnehmbar ist. Dort ist das Behandlungselement 30, 30' jeweils im auf das Pfostenteil 2 aufmontierten Zustand dargestellt. Ebenfalls dargestellt ist dabei ein das Pfostenteil 2 im Bereich seines Außengewindes 6 ringförmig umgebender Raumbereich 36 im Kieferknochen 38, der von Periimplantitis befallen und dementsprechend mit Bakterien belastet ist.

Das Behandlungselement 30, 30' weist einen im Wesentlichen als Zylindermantelkörper ausgeführten Grundkörper 40 auf, der mit seiner die Kontaktfläche 32 bildenden Stirnfläche 42 auf die obere Stirnfläche oder -kante 34 des Pfostenteils 2 aufgesetzt ist. Für eine erhöhte mechanische Stabilität ist zudem an den Grundkörper 40 ein Verbindungszapfen 43 angeformt, der hinsichtlich Kontur und Geometrieparameter an den Aufnahmekanal 12 im Pfostenteil 2 angepasst und in diesen einschiebbar ist, so dass eine zuverlässige Positionierung und vorübergehende Fixierung bei eingeschobenem Verbindungszapfen ermöglicht ist.

Im Innenraum des Grundkörpers 40 und koaxial mit diesem ist ein zentraler Innenkanal 44 vorgesehen. Das Behandlungselement 30 in der Variante gem. FIG. 6 ist zur Nutzung der Schraubverbindung während der vorgesehenen Behandlungsphase ausgelegt, und dementsprechend ist in dieser Variante im Innenkanal 44 eine Verbindungsschraube 46 geführt. Die Verbindungsschraube 46 greift mit ihrem Schraubengewinde 48 in das innerhalb des Pfostenteils 2 vorgesehene Schraubgewinde 20 ein. Anders als die für die Verbindung des eigentlichen Abutments 4 mit dem Pfostenteil 2 vorgesehene Verbindungsschraube 18 ist die Verbindungsschraube 46 nicht für eine hohe mechanische Belastbarkeit und Langlebigkeit der hergestellten Schraubverbindung ausgelegt; vielmehr liegen für die Verbindungsschraube 46 andere Auslegungskriterien zugrunde. Dabei ist insbesondere der nachfolgend erläuterte Behandlungsablauf berücksichtigt, bei dem die Verbindungsschraube 46 und mit dieser das Pfostenteil 2 als Elektrode für die Strompulse dienen sollen. Demzufolge ist die Verbindungsschraube 46 aus elektrisch leitfähigem Material, insbesondere aus einem Metall wie beispielsweise Titan, gefertigt.

In der - ansonsten im Wesentlichen baugleichen - Variante gem. FIG. 7 ist das Behandlungselement 30' demgegenüber für eine Steckverbindung mit dem Pfostenteil 2 ausgelegt. Dazu ist in dieser Variante im Innenkanal 44 ein elektrisches Kontaktierelement 49 geführt und vorzugsweise in ihrer Längsrichtung verschiebbar gelagert. Für die Auslegung des elektrischen Kontaktierelements 49 und seine räumliche Konfigurierung ist ebenfalls der nachfolgend erläuterte Behandlungsablauf berücksichtigt, bei dem das elektrische Kontaktierelement 49 und mit diesem das Pfostenteil 2 als Elektrode für die Strompulse dienen sollen. Demzufolge ist in dieser Variante das elektrische Kontaktierelement 49 aus elektrisch geeignet leitfähigem Material, insbesondere aus einem Metall wie beispielsweise Titan, gefertigt und derart im Innenkanal 44 gelagert und geführt, dass es in vollständig in den Innenkanal 44 eingeschobenem Zustand mit seiner Nadelspitze 50 an den Boden 52 oder an einem anderen Bereich des Innenkanals 44 anstößt und somit einen zuverlässigen elektrischen Kontakt mit dem Pfostenteil 2 herstellt. Alternativ kann das elektrische Kontaktierelement 49 in seinem Endbereich auch als spreizbares federndes Element ausgeführt sein, das eine zuverlässige elektrische Kontaktierung an den seitlichen Innenflächen des Innenkanals 44 herstellt.

Das Behandlungselement 30, 30' ist zur Zuführung von Reinigungsflüssigkeit, die unter anderem auch das Abtöten von Keimen oder Bakterien bewirken kann, in den Raumbereich 36 ausgelegt. Dazu ist der Grundkörper 40 mit einer Anzahl von Medienkanälen 56 versehen, die eingangsseitig mit einem Versorgungs- oder Bespeisungssystem für die Behandlungsflüssigkeit verbunden ist. Im Ausführungsbeispiel sind die Medienkanäle 56 dabei durch in einen den Grundkörper 40 umgebenden Ringkörper 58 eingebrachte Nuten 59 gebildet. Der Ringkörper 58 ist dabei auf den Grundkörper 40 aufgeschoben, so dass die Nuten 59 nach innen hin durch den Außenmantel des Grundkörpers 40 abgeschlossen sind und damit ein Kanalsystem aus den Medienkanälen 56 bilden. Alternativ könnten die Medienkanäle natürlich auch auf andere Weise direkt in den Grundkörper 40 eingebracht sein.

In unmittelbarer Nähe zum Kontaktbereich der Stirnfläche 42 des Grundkörpers 40 mit der Stirnkante 34 des Pfostenteils 2 weist das durch die Medienkanäle 56 gebildete Kanalsystem eine Anzahl von Auslassöffnungen 60 auf, von denen der Übersichtlichkeit halber in den FIG. 6, 7 jeweils lediglich zwei gezeigt sind. Jeder Medienkanal 56 ist dabei im Ausführungsbeispiel mit einer Auslassöffnung 60 versehen. In Querschnitt und Anzahl können die Auslassöffnungen 60 aber auch an individuelle Vorgaben angepasst sein. Beispielsweise könnte eine einzige Auslassöffnung vorgesehen sein, die beispielsweise einen vollständig umlaufenden Ringspalt zwischen der Stirnfläche 42 und der Stirnkante 34 bildet. Alternativ kann auch eine Mehrzahl von Auslassöffnungen 60 vorgesehen sein, die insbesondere in Umfangsrichtung des Grundkörpers 40 gesehen gleichmäßig um dessen Umfang herum angeordnet sein können. Alternativ kann auch lediglich ein einziger Medienkanal 56 mit einer zugeordneten einzelnen Auslassöffnung 60 vorgesehen sein, die bevorzugt individuell positionierbar und somit zur lokalisierten Ausbringung von Behandlungsflüssigkeit in einen begrenzten Raumbereich ausgestaltet sein kann.

Das durch den oder die Medienkanäle 56 gebildete Kanalsystem mündet mit seinen Auslassöffnungen 60 unmittelbar benachbart zur Stirnfläche 42 und damit unmittelbar oberhalb des Raumbereichs 36, so dass das aus den Auslassöffnungen 60 ausströmende Medium mehr oder weniger direkt in den darunterliegenden Raumbereich 36 gelangt. Durch diese für sich als eigenständig erfinderisch angesehene Ausgestaltung des Grundkörpers 40 bildet das Behandlungselement 30, 30' somit ein Kanalsystem, mit dem eine Behandlungsflüssigkeit gezielt und wirksam direkt in den behandlungsbedürftigen Raumbereich 36 eingebracht werden kann.

Zusätzlich ist das Behandlungselement 30, 30' noch als elektrisches System spezifisch ausgestaltet. Dabei ist als Auslegungsgrundsatz insbesondere vorgesehen, eine gepulste Beaufschlagung des in den Medienkanälen 56 geführten Mediums, insbesondere der darin geführten Kochsalzlösung, mit Strompulsen zu ermöglichen. Das Behandlungselement 30, 30' ist dabei dafür ausgelegt, den zu Reinigungszwecken des inserierten Implantat-Teils 2 vorgesehenen Stromfluss gezielt lokalisiert im behandlungsbedürftigen Raumbereich 36 aufbringen zu können. Das Behandlungselement 30, 30' ist dabei nach dem Auslegungsprinzip aufgebaut, dass der elektrische Strom dem inserierten Implantat-Teil 2 zugeführt und dieses als Elektrode verwendet werden kann. Dazu umfasst das Behandlungselement 30, 30' ein erstes, einen elektrischen Strompfad bildendes und über die Verbindungsschraube 46 bzw. das elektrische Kontaktierelement 49 mit dem Implantat-Teil 2 elektrisch verbundenes Leitungselement 62, das seinerseits an eine geeignet gewählte Strom- oder Spannungsquelle anschließbar ist.

Zur Bildung eines Gegenpols oder der Gegenelektrode ist die Nutzung der elektrischen Leitfähigkeit der in den Medienkanälen 56 geführten Reinigungsflüssigkeit vorgesehen. Dazu ist der Innenraum der Medienkanäle 56 seinerseits elektrisch mit dem anderen Pol der Strom- oder Spannungsquelle verbunden. Damit bilden die Auslassöffnungen 60 der Medienkanäle 56 in elektrischer Hinsicht einen Kontakt 64 oder einen elektrischen Kontaktpunkt, über den der Stromfluss in das Implantat-Teil 2 oder vom Implantat-Teil 2 erfolgt. Durch diese Nutzung der in unmittelbarer Nachbarschaft zum behandlungsbedürftigen Raumbereich 36 positionierten Auslassöffnungen 60 als elektrischen Kontakt 64 ist erreicht, dass der zu Behandlungs- und Reinigungszwecken angelegte elektrische Strom durch die von den Bakterien befallene Oberflächenzone des inserierten Implantat-Teils 2 hindurch- und von dort aus weitgehend unmittelbar, also insbesondere ohne "Umwege" über weiteres Körpergewebe oder dergleichen, zur Kontaktfläche 64 bzw. zu dem Kontaktpunkt fließen kann. Die Medienkanäle 56 einschließlich der darin geführten, elektrisch leitfähigen Reinigungsflüssigkeit und den entsprechenden Anschlusselementen bildet somit im Ausführungsbeispiel ein zweites, einen elektrischen Strompfad zu dem endseitig angeordneten Kontakt 64 bildendes Leiterelement 66.

Alternativ könnte das zweite Leiterelement 66 aber auch in der Art einer "konventionellen" Elektrode, also insbesondere als elektrisch leitfähiges nadelartiges Element aus Metall, ausgeführt sein. Diese könnte insbesondere in einer Längsrichtung im Wesentlichen parallel zur Zentralachse des Grundkörpers 40 verschiebbar an diesem gelagert sein. Zur Bildung dieser Elektrode oder auch einer bedarfsweise zusätzlich vorgesehenen dritten Elektrode, die beispielsweise zur lokalen Erzeugung eines elektrischen Feldes beispielsweise zur Feldverstärkung vorgesehen sein kann, kann gegebenenfalls noch ein geeignet geformter weiterer Metallkörper 68 vorgesehen sein. Dabei kann das Behandlungselement 30, 30' auch ohne die Medienkanäle ausgeführt sein, wobei die Gegenelektrode und damit der zweite Strompfad ausschließlich über den Metallkörper 68 gebildet sein kann. In diesem Fall wird der Kontakt 64 über die endseitig freie Fläche des jeweiligen Elektrodenkörpers gebildet.

Durch die Positionierung der Auslassöffnungen 60 und/oder der endseitigen Kontaktfläche 69 des Metallkörpers 68 ist zudem sichergestellt, dass die durch sie gebildete Kontaktfläche 64 des zweiten Leiterelement 66 in seitlicher Richtung gesehen in einem Abstand von mindestens 1 mm und von höchstens 10 mm von der zentralen Längsachse des Dental-Implantat-Teils 2 positioniert ist.

Der Grundkörper 40 des Behandlungselements 30, 30' könnte aus isolierendem Material wie beispielsweise einer Keramik oder einem Kunststoff gebildet sein. Im Ausführungsbeispiel ist er jedoch aus Metall, nämlich aus Titan, gefertigt. Um dabei eine zuverlässige elektrische Isolierung der Komponenten gegeneinander zu gewährleisten, ist er an seiner die Kontaktfläche zum Dental-Implantat-Teil 2 bildenden Stirnfläche 42 mit einer isolierenden Beschichtung 70 versehen und somit elektrisch isoliert ausgeführt. Des Weiteren ist der Ringkörper 58 aus isolierendem Material wie beispielsweise einer Keramik ausgeführt.

In einer alternativen, nicht näher dargestellten Ausführungsform kann auch eine nicht dichtende Verbindung beim Aufsetzen des Grundkörpers 40 auf die Stirnfläche 34 des Pfostenteils 2 vorgesehen sein. Dies ermöglicht während der Behandlungsphase ein Eindringen der vorgesehenen Behandlungs- oder Reinigungsflüssigkeit in den Innenraum des Pfostenteils 2, so dass dieser bei Bedarf ebenfalls einer Reinigung unterzogen werden kann.

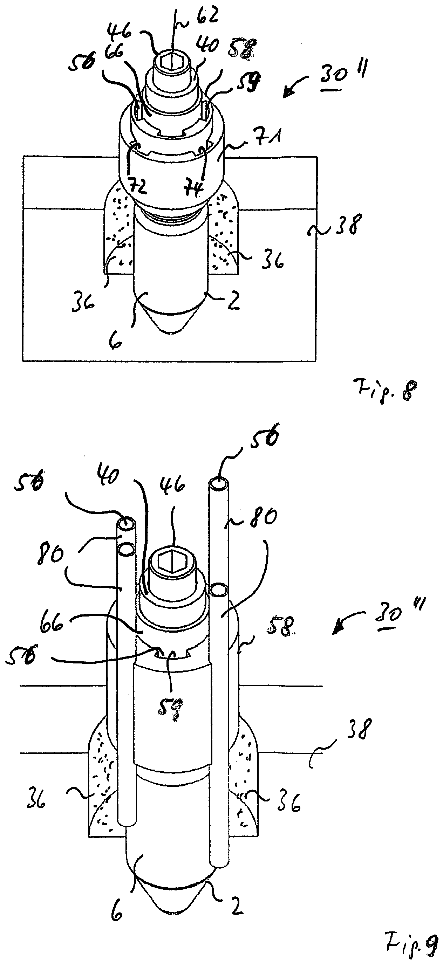

In einer alternativen Ausführungsform ist das Behandlungselement 30", wie dies in perspektivischer Ansicht in FIG. 8 gezeigt ist, mit einem weiteren Kanalsystem versehen, dass beispielsweise als Rückführkanal für die Reinigungsflüssigkeit, als separate Zuleitung zur Einbringung eines Mediengemischs oder auch als Absaugkanal vorgesehen sein kann. Dazu ist in dieser Ausführungsform der Ringkörper 58 von einem weiteren Ringkörper 71 umgeben, in den zur Bildung weiterer Medienkanäle 72 innenseitig ebenfalls Nuten 74 eingebracht sind.

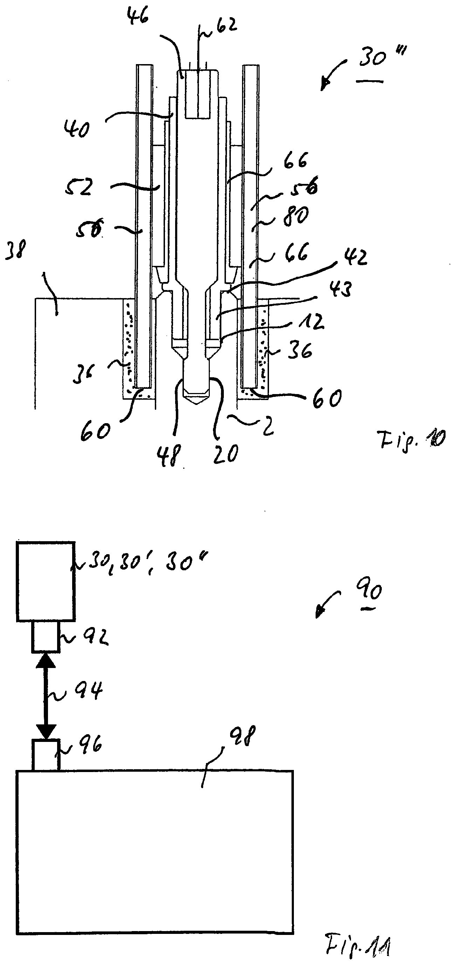

In den vorstehend erläuterten Ausführungsformen sind die Medienkanäle 56 und/oder die Leiterelemente 60, 66 in im Wesentlichen integrierter Bauweise ausgeführt und innerhalb des Grundkörpers 40 bzw. der mit diesem verbundenen Ringkörper 58, 71 geführt. Alternativ oder zusätzlich können aber auch einige oder alle der Medienkanäle 56 und/oder der Leiterelemente 60, 66 außerhalb am Grundkörper 40 angeordnet und über geeignete Haltesysteme mit diesem verbunden sein. Diese Ausführungsform ist im Ausführungsbeispiel in perspektivischer Ansicht gemäß FIG. 9 und im Längsschnitt gemäß FIG. 10 gezeigt. Zusätzlich zu den bereits erläuterten Komponenten ist das dort gezeigte Behandlungselement 30'" mit außen am Ringkörper 58 angeordneten, in Längsrichtung verschiebbaren Kanalelementen 80 versehen. Diese können analog zu den Medienkanälen 56 in der Art von Kanülen oder dergleichen ausgeführt sein und mit Reinigungsflüssigkeit beaufschlagt werden und zusätzlich als Leiterelement 66 dienen. Alternativ können sie aber auch metallisch in der Art von Elektroden ausgeführt sein und elektrisch geeignet mit der Strom- oder Spannungsquelle verbunden werden. Zusätzlich ist im Ausführungsbeispiel gem. FIG. 9 noch eine Variante gezeigt, bei der zusätzlich zu den von den extern angeordneten Kanalelementen 80 gebildeten Medienkanälen auch noch durch Nuten 59 im Ringkörper 58 gebildete integrierte Medienkanäle 56 vorgesehen sind.

Das Behandlungselement 30, 30', 30", 30"' wird bevorzugt in einem Behandlungssystem 90 verwendet, wie es in FIG. 10 gezeigt ist. Das Behandlungssystem 90 ist für ein inseriertes Dental-Implantat-Teil oder Pfostenteil 2 vorgesehen und umfasst das Behandlungselement 30, 30', 30",30'" und zusätzlich zu diesem ein Verbindungselement 92 zwischen diesem und einem Schlauchpaket 94, eine Steckverbindung 96 zwischen diesem und einer außerhalb des Patientenmundes angeordneten Versorgungs- und Steuereinheit 98. Diese Versorgungs- und Steuereinheit 98 beinhaltet eine elektrische Versorgung, welche zwischen der Elektrode im Pfostenteil 2 und einer weiteren Elektrode, welche sich in dem Behandlungselement 30, 30', 30", 30"', den Steckverbindungen 96, dem Schlauchpaket 94 und/oder der Versorgungs- und Steuereinheit 98 befinden kann, eine Spannung anlegen kann und/oder einen Strom fließen lassen kann. Diese Elektrode weist eine elektrisch leitende Kontaktstelle zu den Medien/Elektrolyten aufweisen.

Diese Spannung oder dieser Strom kann als Gleichspannung/-strom, von der Polarität in beide Richtungen oder als Wechselspannung an die beiden Elektroden angelegt werden. Handelt es sich um eine Wechselspannung, kann diese als Sinus, Dreieck, Rechteck oder jede erdenklich Übereinanderlagerung dieser mit unterschiedlichen Frequenzen sein. Darüber hinaus kann diese Wechselspannung von einer Gleichspannung überlagert sein. Es besteht auch die Möglichkeit, eine pulsierende Gleichspannung zu verwenden. Zur Erzeugung eines elektrischen Feldes kann eine dritte, elektrisch isolierte Elektrode vorzugsweise im Behandlungselement 30, 30', 30", 30'" angebracht sein.

Über dies hinaus beinhaltet die Versorgungs- und Steuereinheit 98 Vorratsbehälter für mindestens zwei Flüssigkeiten oder Elektrolyte. Diese können über Pumpen und über eine oder mehrere Ventile oder Ventileinheiten gleichzeitig (mischend) oder nacheinander über das Schlauchpaket 94 in das Behandlungselement 30, 30', 30" gefördert werden. In einem besonders günstigen Fall beinhaltet die Versorgungs- und Steuereinheit 98 auch eine Absaugung, welche die über das Behandlungselement 30, 30', 30", 30'" zugeführten Flüssigkeiten oder Elektrolyte nach ihrem Einsatz wieder absaugen zu können. Die Versorgungs- und Steuereinheit 98 beinhaltet in einer besonders günstigen Ausführung zudem eine CO₂ Aufbereitung für Wasser oder andere Flüssigkeiten/Elektrolyte. Zur Prozessoptimierung kann in die Versorgungs- und Steuereinheit 98 auch eine Medientemperierung integriert sein.

Das Schlauchpaket 94 und die Steckverbindungen 96 sind so ausgelegt, dass sie den Stromfluss und den Medienfluss gewährleisten können. Bei einer vollständigen Ausstattung wären das insbesondere drei elektrische Leiter und zwei Flüssigkeits-/Elektrolytleiter.

Das Elektrodenmaterial kann aus dem gleichen Material wie das Pfostenteil 2 sein. Da die Pfostenteile 2 bevorzugt aus Titan oder einer Titanlegierung hergestellt sind, ist es bevorzugt, die weitere/weiteren Elektroden aus einem anderen Metall auszuführen. Titan und dem Titan ähnliche Metalle bilden bei der anodischen Bestromung meist eine schützende Oxidschicht, welche als Isolator fungiert. Um bei kathodischer Bestromung des Pfostenteils 2 nicht über eine solche Oxidschicht den Stromfluss einzuschränken, ist es vorteilhaft, ein nicht oder kaum eine Oxidschicht bildendes Metall als Gegenelektrode zu verwenden. In einem besonders günstigen Fall korrodiert diese Elektrode weder bei Kontakt mit den Medien/Elektrolyten noch bei der Beaufschlagung mit einer Spannung oder einem Strom. Vorzugsweise ist diese Elektrode aus Gold, Platin, Palladium ausgeführt.

Sollte auch das Innere des inserierten Implantates/Pfostenteils 2 kontaminiert sein und folglich gereinigt werden, besteht die Möglichkeit das Innere separat oder gemeinsam mit dem Medium zu spülen und mit Strom zu beaufschlagen.

Die Leiterelemente können auch in Form einer flexiblen oder festen Membrane ausgestaltet seine, welche keine Flüssigkeiten passieren lässt sondern lediglich in dem Elektrolyten vorhandene Ionen. Bei einer solchen Ausgestaltung mündet vorzugsweise einer der Strompfade im Inneren des Pfostenteils 2 und führt weiter an den in diesem Fall gar nicht oder nur teilweise abdichtenden Kontaktflächen 32 vorbei bis an die äußere Oberfläche des Pfostenteils 2.

Die für die Verfahrensführung vorgesehene Behandlungsflüssigkeit ist in besonders vorteilhafter Ausgestaltung im Hinblick auf die vorgenannten Aspekte geeignet gewählt und zusammengesetzt. Die Auswahl und Zusammensetzung der Grundbestandteile der Behandlungsflüssigkeit ist dabei insbesondere im Hinblick auf die beabsichtigte Wirkungsweise, d. h. der Aufbringung eines elektrischen Stroms im Raumbereich der behandlungsbedürftigen Oberfläche, vorgenommen, wobei insbesondere sichergestellt ist, dass eine für diesen Zweck ausreichend hohe elektrische Leitfähigkeit in der Behandlungsflüssigkeit vorliegt. Diese soll insbesondere durch eine ausreichend hoch gewählte Ionendichte in der Behandlungsflüssigkeit sichergestellt werden. Dazu ist als ein Grundbestandteil der Behandlungsflüssigkeit ein Metallsalz vorgesehen, bevorzugt in wässriger Lösung. Dieses liefert die Ionen für den Stromtransport, und zudem können die nach der jeweiligen Elektrodenreaktion entstehenden Umsetzungsprodukte auch noch geeignete biochemische Wirkungen aufweisen. Durch die gezielte Wahl einer ausreichend hohen elektrischen Leitfähigkeit soll bei einer Durchführung des Reinigungsverfahrens am inserierten Implantat sichergestellt werden, dass der Stromfluss durch die Behandlungsflüssigkeit und damit durch die behandlungsbedürftigen Teile und Komponenten, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit der Behandlungsflüssigkeit sollte dabei möglichst ein Vielfaches der elektrischen Leitfähigkeit von Blut, Knochen, Weichgewebe, Fettgewebe oder anderen Körpermaterialien aufweisen.

Demzufolge werden bei der Auswahl und Zusammensetzung der Grundbestandteile für die Behandlungsflüssigkeit insbesondere folgende Leitfähigkeitswerte berücksichtigt (die elektrische Leitfähigkeit σ wird dabei in der üblichen Einheit mS/cm angegeben):

| | |
|---|---|
| Haut: | 0,03 - 0.1 mS/cm |
| Knochen: | 0,06 - 0,2 mS/cm |
| Fettgewebe: | 0,20 - 1,0 mS/cm |
| Muskelgewebe: | 0,80 - 2,5 mS/cm |
| Blut: | ca. 6,7 mS/cm |
| andere Körperflüssigkeiten: | ca. 15 mS/cm |

Um das Gefährdungspotential für den Patienten geeignet gering zu halten und den Stromfluss auf die erwünschten Regionen zu begrenzen, sollte die elektrische Leitfähigkeit daher mindestens das Doppelte, bevorzugt das Fünffache, besonders bevorzugt das Zehnfache der Leitfähigkeit sonstiger Körperflüssigkeiten betragen. Daher sollte die elektrische Leitfähigkeit der Behandlungsflüssigkeit einen Wert von mindestens 30 mS/cm, vorzugsweise mindestens 75 mS/cm und besonders bevorzugt mindestens 150 mS/cm aufweisen. Im Vergleich zu Blut bedeutet dies, dass die elektrische Leitfähigkeit der Behandlungsflüssigkeit vorzugsweise mindestens etwa das Fünffache, bevorzugt mindestens etwas das Zehnfache und besonders bevorzugt mindestens etwa das Zwanzigfache der Leitfähigkeit von Blut beträgt. Messungen haben ergeben, dass beim Einsatz einer solchermaßen gewählten Behandlungsflüssigkeit die elektrische Spannung, der das Körpergewebe, das Blut, die Körperflüssigkeiten etc. ausgesetzt werden, geringer als 6 V, vorzugsweise geringer als 3 V, besonders bevorzugt kleiner als 1,5 V ist. Damit können Schädigungen für den Patienten aufgrund der gering gehaltenen Spannungen sicher ausgeschlossen werden. Zur Einhaltung einer solchen Leitfähigkeit ist insbesondere die lonenkonzentration in der Behandlungsflüssigkeit und in den sie bildenden Grundbestandteilen ausreichend hoch gewählt; hierfür können Laugen, Säuren, Salze und/oder andere ionenbildende Stoffe oder Stoffverbindungen zum Einsatz kommen.

Bei der Auswahl und Zusammensetzung der Grundbestandteile der Behandlungsflüssigkeit ist in besonderem Maße berücksichtigt, dass die reinigende oder biofilmablösende Wirkung der elektrolytischen Behandlung einer kontaminierten Implantatoberfläche auf einer Kombination mehrerer Ursachen beruht, die möglichst ergänzend zueinander nutzbar gemacht werden sollten. Zum einen können sich beim Stromfluss durch den Elektrolyten bevorzugt im Bereich der Elektroden Gase oder Gasblasen bilden, welche eine abhebende (mechanische) Wirkung auf den Biofilm haben. Die Entstehung dieser Gase erfolgt unmittelbar an der als Elektrode dienenden Implantatoberfläche und somit zwischen dieser und dem Biofilm. Die dabei entstehenden Gasblasen beeinflussen mit ihrer Wachstumsgeschwindigkeit und ihrer maximalen Größe den Ablösungsprozess.

Als zweite Ursache für die das Implantat reinigende oder den Biofilm ablösende Wirkung des elektrolytischen Prozesses ist die zersetzende, zerstörende und auflösende Wirkung der elektrolytisch entstehenden Stoffe oder Stoffverbindungen auf die eigentliche Anhaftung des Biofilms an der Implantatoberfläche, also auf den Klebe- oder Verankerungsmechanismus, zu nennen.

Die dritte Ursache für die reinigende oder ablösende Wirkung des elektrolytischen Prozesses basiert auf materialabtragenden Effekten des Implantatmaterials, wobei Bestandteile oder Partikel des eigentlichen Implantats in dessen Oberflächenbereich aus diesem herausgelöst werden.

Die vierte Ursache für die reinigende oder ablösende Wirkung des elektrolytischen Prozesses basiert auf der Oxidschichtbildung metallischer Implantate, welche dies erlauben. Hierbei durchdringen Metallatome des metallischen Grundmaterials die evtl. schon vorhandene Oxidschicht basierend auf der angelegten elektrischen Spannung und reagieren mit Stoffen des Elektrolyts (meist Sauerstoff => Metalloxidbildung). Bei Metallen, welche keine Oxidschicht bzw. keine mechanisch stabile Oxidschicht bilden, können auch nicht-oxydische Stoffverbindungen entstehen (meist Salze), die dann in Lösung gehen.

Die für die Bildung der Behandlungsflüssigkeit vorgesehenen Grundbestandteile sind im Hinblick auf diese Effekte geeignet gewählt und miteinander kombiniert. Zudem ist als grundlegendes Auslegungsziel berücksichtigt, dass keine toxischen oder anderweitig einen Patienten gefährdenden oder für ihn unangenehmen Effekte auftreten sollten, so dass die Behandlungsflüssigkeit auch für einen Einsatz am inserierten Dentalimplantat, also im Patientenmund, geeignet ist. Im Ausführungsbeispiel sind dabei als Grundbestandteile mindestens ein Salz einerseits und eine Säure andererseits, bevorzugt verdünnt mit Wasser, vorgesehen, deren Auswahl und Zusammensetzung sich insbesondere nach den genannten Kriterien richtet. Besonders bevorzugt ist dabei als Säure Phosphorsäure, Zitronensäure, Äpfelsäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium- oder Kaliumiodid, -chlorid, -nitrat, carbonat oder -hydrogencarbonat und/oder Ammoniumchlorit, -nitrat oder -iodid oder eine Kombination von diesen vorgesehen.

Des Weiteren ist dabei berücksichtigt, dass der vorgesehene elektrolytische Prozess wahlweise mit anodischer oder mit kathodischer Schaltung des Pfostenteils geführt werden kann. Demzufolge wird im Folgenden in eine Anodenreaktion und eine Kathodenreaktion unterteilt.

Bei einer Anodenreaktion, also bei anodischer Schaltung des Pfostenteils 2, wer-den an der Anode die in der Behandlungsflüssigkeit vorhandenen Anionen generell durch den Entzug von Elektronen oxidiert. Dabei kann es zu einer unmittelbaren Reaktion mit dem Material kommen, insbesondere zur Bildung einer Oxidschicht und/oder eines Salzes mit dem Implantatmaterial. Knochenimplantate und dementsprechend auch das Pfostenteil 2 bestehen meist aus Titan, Zirkonium, Tantal oder aus Legierungen dieser Metalle. Darüber hinaus werden noch andere Metalle hinzulegiert. Diese Metalle oder Metalllegierungen weisen meist einen hohen Grad an Oxidschichtbildungen auf. Diese Oxidschichtbildung hat eine passivierende Wirkung auf die Oberfläche. Die Folge darauf ist die Unterbindung oder zumindest sehr starke Reduzierung der Anodenreaktion dieser Metalle oder Metalllegierungen. Da sich im Biofilm meist Stoffverbindungen mit Sauerstoff befinden, ist eine Unterbindung dieser Passivierung meist nicht möglich. Sollte das Pfostenteil anodisch geschaltet sein, so ist der ablösende reinigende Effekt somit meist auf die Oxidschichtbildung beschränkt. Bei höheren Betriebsspannungen von beispielsweise mehr als 10 V konnte bei umfangreichen Untersuchungen zwar aufgezeigt werden, dass ein materialabtragender Prozess möglich ist, dass dieser aber mit einer starken Wärmeentwicklung verbunden ist. Diese Wärmeentwicklung kann zur unerwünschten Nekrose des Knochens führen. Darüber hinaus verändert der damit einhergehende Materialabtrag auch in unerwünschter Weise die Eigenschaften der ursprünglichen Implantatoberfläche.

Als Ausnahme hierzu hat sich überraschend gezeigt, dass bei einem Basismaterial des Pfostenteils 2, bei dem Aluminium als Legierungsbestandteil vorhanden ist (beispielsweise bei Titan grade5, das etwa 6 % Aluminiumanteil und 4 % Vanadiumanteil aufweist) auch eine anodische Bestromung des Pfostenteils 2 möglich ist, ohne dass die Bildung einer Oxidschicht den Prozess zu sehr behindert. Hierdurch kann, je nach Zusammensetzung der Behandlungsflüssigkeit, Chlor- oder lodgas oder auch CO2 direkt an der Oberfläche des Pfostenteils 2 erzeugt und somit unmittelbar für die angestrebte Ablösung des Biofilms nutzbar gemacht werden. Für eine derartige Verfahrensführung ist das Behandlungselement 30 besonders vorteilhaft mit einer leitfähigen Oberflächenbeschichtung versehen, beispielsweise aus DLC ("diamond-like carbon"), einem Metall, leitfähigem Kunststoff oder einer elektrisch leitfähigen Keramik.

Als besonders vorteilhaft hat sich dabei herausgestellt, dass bei einem Basismaterial Titan grade IV oder Titan grade V des Pfostenteils durch Zugabe von CO2 in die Behandlungsflüssigkeit trotz sich bildender Oxidschicht bei anodischer Bestromung eine CO2-Bildung, Cl-Bildung und/oder I-Bildung möglich ist, die einen länger andauernden Stromfluss ermöglicht.

Aus den genannten Gründen ist das Pfostenteil 2 bei der Behandlung mit der Behandlungsflüssigkeit jedoch im Allgemeinen bevorzugt kathodisch geschaltet. Dabei wandern positiv geladene Ionen (Kationen) zur Oberfläche des Pfostenteils 2. Dies können insbesondere H+-Ionen, Metallionen oder langkettige Kohlenwasserstoffionen, z. B. aus ionischen Flüssigkeiten, sein. Das als Grundbestandteil für die Behandlungsflüssigkeit vorgesehene Salz ist dabei insbesondere gezielt im Hinblick auf die Eigenschaften der Kationen ausgewählt, die den genannten Prozess begünstigen oder überhaupt ermöglichen sollen. Zur Erzeugung einer möglichst hohen elektrischen Leitfähigkeit eignen sich insbesondere kleine Ionen (H+-Ionen oder Metallkationen), die zudem in der Art eines weiteren besonders günstigen Effekts den gegebenenfalls vorhandenen Biofilm vergleichsweise leicht durchdringen können. H+-Ionen werden an der durch das Pfostenteil 2 gebildeten Kathode zu elementarem Wasserstoff H reduziert. Dies erzeugt eine Blasenbildung. Eine weitere Kathodenreaktion ist das Abscheiden von elementaren Metallen. Die meisten Metalle würden sich aber als flächendeckender Metallniederschlag abscheiden. Dies wäre ein unerwünschter Effekt, da diese Beschichtung ungünstige Haftungseigenschaften und ungünstige chemische (elektrochemische und biochemische) Eigenschaften hätte.

Aus den genannten Gründen sind für die Kathodenreaktion an der Implantatoberfläche bevorzugt Metalle vorgesehen, deren Kationen kein biologisches Gefährdungspotential für den Patienten darstellen, und die in elementarem Zustand eine möglichst starke chemische Reaktion mit Wasser im Elektrolyten ausüben. Alkalimetalle, Edelmetalle und/oder Aluminium reagieren unmittelbar mit der elektrolytischen Reduzierung an der Kathode mit dem umgebenden Wasser zu elementarem Wasserstoff und seinem Metallkationen und OH--Ionen. Dies bedeutet, dass sich Wasserstoffblasen und das Hydroxid des verwendeten Metallions bilden. Durch die Kombination dieser Komponenten ist somit neben der ablösenden Wirkung des entstehenden Wasserstoffs erreicht, dass das Metallhydroxid antibakteriell wirkt und einen verdünnenden oder auflösenden Einfluss auf den Biofilm bzw. dessen Adhäsionsmechanismus ausübt.

Um Unverträglichkeiten mit dem Körpergewebe zu vermeiden, sind als Metallkationen insbesondere die körpereigenen (z. B. Kalium- und/oder Natriumionen) besonders bevorzugt. Darüber hinaus eignen sich auch Calcium, Magnesium und/oder Aluminiumionen. Das als Grundbestandteil für die Behandlungsflüssigkeit vorgesehene Salz ist daher besonders bevorzugt ein Salz dieser Metalle, insbesondere da diese Metallkationen ohnehin nur in Form eines Salzes, z. B. in Wasser gelöst, zur Verfügung gestellt werden können.

Diese Metallsalze können Verbindungen der genannten Metalle mit einem geeigneten Salzbildner, beispielsweise mit Schwefel, Phosphor, Stickstoff, Fluor, Chlor, Jod, Brom, Kohlenwasserstoff, Sauerstoff, Bor oder anderen Nichtmetallen, sein. Der Salzbildner wird dabei vorteilhafterweise unter Berücksichtigung des Grundsatzes "je größer das Anion, desto geringer die elektrische Leitfähigkeit" und im Hinblick auf die grundsätzlich gewünschte hohe elektrische Leitfähigkeit geeignet gewählt. Als Anion werden zudem bevorzugt nur Stoffe in Betracht gezogen, welche weder die Gesundheit noch das periimplantäre Gewebe beeinflussen. Weiterhin gilt zu beachten, dass unangenehme Gerüche oder Geschmacksverbindungen unerwünscht sind. Aus diesen Gründen werden Schwefelanionen oder Anionen, die Schwefel in Kombination mit Sauerstoff oder anderen Elementen enthalten, als eher ungeeignet angesehen. Dies gilt auch für Fluor-, Brom-, Stickstoff- und Borionen, gegebenenfalls auch in Kombination mit weiteren Elementen.

Demgegenüber haben Phosphate, Phosphationen und Hydrogenphosphationen meist keine oder kaum eine schädliche Wirkung. Chlorionen oder Ionen, welche Chlor enthalten, haben meist eine antibakterielle Wirkung. Sollte das Chlorion allerdings elektrolytisch oxidiert werden und in Wasser elementar vorhanden sein, so bildet sich dabei Salzsäure und hypochlorige Säure. Dies würde zwar in Kombination mit dem kathodisch entstandenen Hydroxid zu einer Neutralisierung führen. Jedoch hat sich bei Untersuchungen gezeigt, dass das Chlor, welches an der Gegenelektrode zum Implantat (Anode) entsteht, in großem Maße dem Elektrolyten als Gas entweicht. Sollte das Chlor bei der Behandlung nicht restlos abgesaugt werden können, kann es zu starken Verätzungen in der Lunge und/oder den Schleimhäuten kommen. Hierbei gilt es abzuwägen, ob der Nutzen für den Patienten oder dessen Gefährdung größer ist.

Zu den Phosphaten des Aluminiums, des Kaliums, des Natriums, des Calciums oder des Magnesiums ist zudem zu bemerken, dass die Löslichkeit in Wasser so gering ist, dass eine ausreichende elektrische Leitfähigkeit des Elektrolyten nicht gewährleistet ist (diese Phosphate eignen sich allerdings sehr gut als Zusatzstoffe des Elektrolyten zur Pufferung des pH-Wertes). Chloride der vier eben aufgeführten Metalle hätten zwar eine ausreichende Löslichkeit in Wasser und eine gute Reinigungs- und Abtötungswirkung auf den Biofilm, können aber nicht als das Optimum angesehen werden. Bei Nitraten und/oder Nitriten ist eine Gefährdung des Patienten durch die Bildung von NOx-Gasen zu erwarten. Aus diesem Grund ist vom Einsatz von Nitriten oder Nitraten abzuraten.

Im Hinblick auf die genannten Auslegungsziele, insbesondere für eine besonders gute Verträglichkeit für den Patienten, ist in bevorzugter Ausführung als Salzbildner Jod vorgesehen. Besonders vorteilhaft ist dabei, dass Jodsalze des Kaliums und des Natriums auch im menschlichen Körper von Natur aus vorhanden sind. Bei der Oxidation von Jodionen an der Anode entsteht zunächst elementares Jod, welches sich in eine Natriumiodid-/Kaliumiodidlösung lösen kann. Es entsteht dabei eine Jod-Kalium-Jodid-Lösung bzw. eine Jod-Natrium-Jodid-Lösung. Beide Lösungen sind starke Desinfektionsmittel, welche sich in der Humanmedizin bewährt haben.

Reine Natrium- oder Kaliumiodidlösungen oder ein Gemisch aus beiden haben jedoch als möglichen Nachteil die Natrium- und/oder Kaliumhydroxidbildung und den damit verbundenen pH-Wertanstieg zur Folge. Als problematisch könnte nämlich ganz allgemein bei der bereits oben beschriebenen Bildung von Metall-hydroxid einzustufen sein, dass ein Metallhydroxid den pH-Wert des Elektrolyten erhöht. Ein solchermaßen erhöhter pH-Wert und die sich bildende Lauge oder Base des gelösten Metallhydroxids könnten einen unerwünschten Einfluss auf das umliegende Gewebe im Patientenmund und insbesondere den Knochen haben. Auch könnten umliegende Zähne geschädigt werden. Zudem könnte die Bildung von Hydroxiden dazu führen, dass diese sich aufgrund ihrer sehr geringen Wasserlöslichkeit auf dem Pfostenteil 2 oder allgemein auf dem behandlungsbedürftigen Bauteil niederschlagen und damit den weiteren Stromfluss und somit den Prozess insgesamt behindern. Allenfalls bei Verwendung eines Calciumsalzes in der Behandlungsflüssigkeit würde das entstehende Calciumhydroxid, das als Bestandteil des Knochenmaterials vorkommt, in den Knochen integriert werden können; Calcium ist somit besonders bevorzugter Bestandteil des Salzes. Um diese unerwünschten Einflüsse zu kompensieren, weist die Behandlungsflüssigkeit als weiteren Grundbestandteil in der Art eines pH-Puffers oder -Reduzierers die Säure auf.

Die Säure ist dabei ihrerseits gezielt in der Art eines Auslegungskriteriums derart gewählt, dass sie möglichst nicht den Patienten oder das periimplantäre Gewebe gefährdet, sondern zum einen das Hydroxid neutralisiert (und auch den pH-Wert möglichst nicht über 7 steigen lässt), wobei zum anderen die Reaktionsprodukte dem eigentlichen Ziel der Reinigung des Implantatkörpers und der Ablösung des Biofilms dienen sollten. Als Mineralsäuren kommen dazu bevorzugt Phosphorsäuren und/oder Phosphatsäuren in Frage. Diese sollten in ihrer Konzentration aus gesundheitsgefährdenden und/oder knochen-/gewebegefährdenden Gründen auf Höchstwerte von 30 % oder bevorzugt von 10 bis 20 % limitiert sein. Eine besonders bevorzugte Säure, welche auch als Mineralsäure angesehen wird und einen besonders positiven Effekt auf das Gesamtziel der Abtötung und Reinigung hat, ist hingegen die Kohlensäure. Diese ist jedoch in ihrer anwendbaren Menge durch das vergleichsweise geringe Lösungsvermögen in Wasser limitiert.

Organische Säuren stellen demgegenüber ähnlich wie Mineralsäuren pH-Wert-reduzierende und Hydroxid neutralisierende H+-Ionen zur Verfügung. Da sie zu-dem keine oder allenfalls geringfügige Schädigungen im Gewebe erzeugen oder beim Patienten insgesamt hervorrufen, sind derartige organische Säuren als Grundbestandteil für die Behandlungsflüssigkeit ganz besonders bevorzugt. Organische Säuren wären z. B. Alkansäuren, Fruchtsäuren, Carbonsäuren sowie Hydroxylcarbonsäuren. Als besonders geeignete Säuren haben sich α-Hydroxylcarbonsäuren herausgestellt. Insbesondere zeigen die besonders bevorzugten Säuren Milchsäure, Zitronensäure und Äpfelsäure keinerlei gesundheitsschädigende Effekte auf den Patienten generell oder auf das periimplantäre Gewebe. Speziell bei stark mit Biofilm behafteten und kontaminierten Implantaten, auf welchen sich auch Zahnstein gebildet hat, zeigten bereits vergleichsweise geringe Dosierungen an Essigsäure einen guten Reinigungserfolg. Weitere Säuren, welche die Reinigung sowie den bakterientötenden Effekt aufweisen, aber aus gesundheitlichen Gründen nicht unbedenklich sind, wären die Fumarsäure, Gluconsäure, Glycolsäure, Salicylsäure, Mandelsäure, Weinsäure, Oxalsäure und Ameisensäure.

Bei der Neutralisierung des Hydroxidions OH- mit dem entsprechenden H+-Ion einer Säure entsteht zusätzlich das Metallsalz der verwendeten Säure des entsprechenden Metallhydroxids. Der vorgesehene Einsatz der Säure ist somit nicht nur zur Pufferung des pH-Werts vorteilhaft, sondern trägt überdies noch zur Umwandlung des vergleichsweise schlecht wasserlöslichen Hydroxids in vergleichsweise gute wasserlösliche Salze bei und verhindert somit die Abscheidung unerwünschten und für den Prozess hinderlichen Niederschlags auf dem behandlungsbedürftigen Bauteil. Die genannten Salze werden insbesondere bei der Kombination der genannten bevorzugten Materielaien u. a. auch in der Medizin eingesetzt. Bei der Neutralisation des Kalium-, Natrium- und/oder Calciumhydroxids mit Milchsäure bildet sich das Kaliumlaktat (es besitzt eine breitbandige antimikrobielle Wirkung), Natriumlaktat bzw. Calciumlactat. Werden die sich bildenden Hydroxide hingegen mit Zitronensäure neutralisiert, bilden sich Zitrate des Kaliums, Natriums bzw. Calciums. Gerade für Natriumzitrat ist dies besonders vorteilhaft, da dieses die Blutgerinnung verhindert. Dies ist besonders vorteilhaft, da während des Prozesses austretendes, auf der Implantatoberfläche gerinnendes Blut die lonenwanderung zur Implantatoberfläche und damit die Fortsetzung des Behandlungsprozesses insgesamt behindern könnte.

Bei der Neutralisation der Hydroxide mit Apfelsäure entstehen hingegen Malate des jeweiligen Kations, welche ebenfalls günstige Auswirkungen auf den Prozess haben. Bei der Neutralisation der Hydroxide mit Essigsäure entstehen Acetate des Kaliums, Natriums und/oder Calciums, welche ebenfalls einen günstigen Einfluss auf den Prozess haben.

Die Lactate, Zitrate, Malate und/oder Acetate des Kaliums, Natriums und/oder Calciums besitzen alle eine säureregulierende Wirkung und sind so verträglich, dass sie nach den jetzigen EU-Verordnungen für Lebensmittelzusatzstoffe in ihrer Verwendung an keine Mengenlimitierung gebunden sind.

Bei der Verwendung von Säuren in dem Elektrolyten in Kombination mit Jodiden und/oder Chloriden des Natriums, Kaliums, Magnesiums, Aluminiums und/oder Calciums hat sich bei der elektrolytischen Anwendung überraschend herausgestellt, dass durch die direkte Reduzierung des H+-Ionen die Blasenbildung derart positiv beeinflusst wird, dass sich der Biofilm deutlich schneller und besser ablöst. Dabei entsteht mit hoher Erzeugungsrate eine Vielzahl vergleichsweise kleiner Bläschen, die aufgrund ihrer vergleichsweise geringen Größe den Biofilm als Ganzes und nicht nur lokal von der darunterliegenden Oberfläche lösen können. Dadurch wird der Biofilm bevorzugt als Ganzes oder in vergleichsweise großen, zusammenhängenden Stücken statt in einer Vielzahl kleinerer Fragmente abgehoben, was eine deutlich verbesserte Reinigungswirkung bedingt.

Anstelle von Metallkationen können auch Ammoniumkationen verwendet werden. Hierbei besteht allerdings die Gefahr, dass bei dem elektrolytischen Prozess andere Ammoniumverbindungen (z. B. Ammoniak) gebildet werden. Dies stellt eine Gefährdung des Patienten dar und zeigt sich auch durch einen sehr unangenehmen Geschmack und Geruch.

Es wurde bei Versuchen beobachtet, dass sich der Biofilm teilweise in sehr kleinen Fragmenten oder aber in größeren zusammenhängenden Teilen ablöst. Letzteres wird bevorzugt, da damit vergleichsweise großflächig sehr günstige Reinigungsergebnisse erzielt werden können. Untersuchungen haben weiterhin gezeigt, dass der Abtransport des gelösten Biofilms und/oder seiner Fragmente durch eine Schaumbildung an der Implantatoberfläche begünstigt wird. Es hat sich herausgestellt, dass es günstig ist, dass nach dem Einsatz eines Elektrolyten aus den beschriebenen Metallsalzen, Säuren und Wasser, die insbesondere für die Abtötung und Ablösung zuständig sind, ein zweiter Elektrolyt zum Einsatz kommt, welcher zusätzlich eine Schaumbildung im Bereich der Kathode aufweist. Eine solche Schaumbildung kann erreicht werden, indem dem Elektrolyten bevorzugt zusätzlich ein Stoff zugegeben wird, der mindestens drei CH2-Kettenglieder oder mindestens ein CH2-Kettenglied und mindestens eine Kohlenstoffringverbindung aufweist. Hierbei können z. B. Öl und/oder Chlorhexidin zum Einsatz kommen. Darüber hinaus können auch ionische Flüssigkeiten verwendet werden, welche vorzugsweise ein I--, Cl-- und/oder OH--Ionen aufweisen. Da der organische Kationenanteil einer ionischen Flüssigkeit unter Umständen auf der Implantatoberfläche reduziert wird und dort verbleicht, ist es in einer besonders günstigen Ausführung möglich, Knochenwachstumsfaktoren an diesen Kationenanteil anzuhängen.

Werden Chloride und Jodide im richtigen Verhältnis gemischt, kann die störende Chlorgasbildung vermieden werden. An der Anode entsteht:

2J + 5Cl + 6H2O → 10HCl + 2HIO3

Dies bedeutet, dass an der Anode Salzsäure sowie Jodsäure gebildet werden. Diese haben sicherlich eine starke antimikrobielle Wirkung und werden auch beim Zusammentreffen mit dem sich kathodisch bildenden Hydroxid wieder neutralisiert.

Eine ganz besonders bevorzugte Zusammensetzung der Behandlungsflüssigkeit, die im Laborversuch besonders günstige Reinigungseigenschaft zeigte, umfasst eine wässrige Lösung von Natriumiodid (Nal) oder Kaliumiodid (KI) in einem Mischungsverhältnis von mindestens 5, bevorzugt mindestens 10, besonders bevorzugt mindestens 20 g des Salzes pro 30 ml Flüssigkeit (d. h. Wasser, H2O, gegebenenfalls mit CO2 angereichert), durch Zugabe von Milchsäure reduziert auf einen pH-Wert von etwa 2,7 bis 2,9.

Bei der Prozessführung ist eine mittlere Stromdichte am Pfostenteil 2 bzw. am behandlungsbedürftigen Bauteil von mindestens 50 mA/cm2, vorteilhafterweise von mindestens 100 mA/cm2, besonders bevorzugt von mindestens 250 mA/cm2, vorgesehen, wobei diese Stromdichte auf die äußere Oberfläche des Pfostenteils 2 (also ohne Berücksichtigung von oberflächenvergößernden Eigenschaften wie beispielsweise Rauigkeit oder Oberflächenstruktur) bezogen ist. Zur Ablösung des Biofilms hat sich eine durchschnittliche Stromdichte von 50 mA/cm2 bis 300 mA/cm2, vorteilhafterweise von 100 mA/cm2 bis 200 mA/cm2 als besonders günstig erwiesen. Zum Abtransport der Biofilmfragmente sollte die mittlere Strom-dichte vorzugsweise auf den Bereich von 300 mA/cm2 bis 5.000 mA/cm2 oder besonders vorteilhaft von 1.000 mA/cm2 bis 2.000 mA/cm2 erhöht werden.

Mit der Zugabe von H2O2 wird der Sprudeleffekt an der Kathode stark gemindert oder unterbunden. Es kommt zu einer sehr starken H2O-Bildung, die zum Spülen der Oberfläche genutzt werden kann.

### Bezugszeichenliste

- 1: Dental-Implantatsystem
- 2: erstes Implantat-Teil/Pfostenteil
- 4: zweites Implantat-Teil
- 6: Außengewinde
- 8: apikales Ende
- 10: Verbindungszapfen
- 12: Aufnahmekanal
- 14: Indizierungselement
- 16: Endkanalstück
- 18: Verbindungsschraube
- 20: Schraubgewinde
- 30, 30', 30",30"': Behandlungselement/Behandlungsabutment
- 32: Kontaktfläche
- 34: Stirn kante
- 36: Raumbereich
- 40: Grundkörper
- 42: Stirnfläche
- 43: Verbindungszapfen
- 44: Innenkanal
- 45: Distanzstücke
- 46: Verbindungsschraube
- 48: Schraubengewinde
- 49: elektrisches Kontaktierelement
- 50: Nadelspitze
- 52: Boden
- 56: Kanal/Medienkanal
- 58: Ringkörper
- 59: Nuten
- 60: Auslassöffnung
- 62: Leitungselement
- 64: Kontakt
- 66: Leiterelement
- 68: Metallkörper
- 69: Kontaktfläche
- 70: isolierende Beschichtung
- 71: Ringkörper
- 72: Medienkanal
- 74: Nut
- 90: Behandlungssystem
- 92: Verbindungselement
- 94: Schlauchpaket
- 96, 98: Versorgungs- und Steuereinheit

## Patentansprüche

1. Behandlungselement (30, 30', 30", 30"') zur Verwendung mit einem in den Kieferknochen eines Patienten eingebrachten Dental-Implantat-Teil (2), mit einem zur mechanischen Verbindung mit dem Dental-Implantat-Teil (2) vorgesehenen Grundkörper (40), der mit mindestens einem Medienkanal (56) für eine Reinigungsflüssigkeit versehen ist,
wobei
der Grundkörper (40) ein erstes, einen elektrischen Strompfad bildendes und mit dem Dental-Implantat-Teil (2) verbindbares Leiterelement (62) und elektrisch isoliert von dem ersten Leiterelement (62) ein zweites, einen elektrischen Strompfad zu einem endseitig angeordneten Kontakt (64) bildendes Leiterelement (66) trägt, dessen Kontakt (64) in einem Raumbereich (36) in der Nähe des Dental-Implantat-Teils (2) positionierbar ist, **dadurch gekennzeichnet, dass** das zweite Leiterelement (66) von einem oder einigen der Medienkanäle (56) gebildet ist.

2. Behandlungselement (30, 30', 30") nach Anspruch 1, dessen Grundkörper (40) zur mechanischen Verbindung mit dem Dental-Implantat-Teil (2) ein an dieses angepasstes Verbindungssystem aufweist.

3. Behandlungselement (30, 30', 30") nach einem der Ansprüche 1 oder 2, dessen zweites Leiterelement (66) als Elektrode oder Kanalelement (80) ausgeführt ist, die bzw. das in einer Längsrichtung im Wesentlichen parallel zur Zentralachse des Grundkörpers (40) verschiebbar an diesem gelagert ist.

4. Behandlungselement (30, 30', 30") nach einem der Ansprüche 1 bis 3, bei dem das zweite Leiterelement (66) derart am Grundkörper (40) angebracht ist, dass sein endseitig angeordneter Kontakt (64) in seitlicher Richtung gesehen in einem Abstand von höchstens 10 mm, vorzugsweise von höchstens 5 mm, von der zentralen Längsachse des Dental-Implantat-Teils (2) positionierbar ist.

5. Behandlungselement (30, 30', 30") nach einem der Ansprüche 1 bis 4, dessen Grundkörper (40) an seiner Kontaktfläche (42) zum Dental-Implantat-Teil (2) elektrisch isoliert ausgeführt ist.

6. Behandlungselement (30, 30', 30") nach einem der Ansprüche 1 bis 5, dessen Verbindungssystem eine Verbindungsschraube (46) zur Einbringung in den Schraubenkanal des Pfostenteils (2) eines zwei- oder mehrteilig ausgeführten Dental-Implantatsystems (1) umfasst.

7. Behandlungselement (30, 30', 30") nach Anspruch 6, bei dem die Verbindungsschraube (46) elektrisch leitend mit dem ersten Leiterelement (62) verbunden ist.

8. Behandlungssystem (90) für ein Dental-Implantat-Teil (2) mit einem Behandlungselement (30, 30', 30") nach einem der Ansprüche 1 bis 7, dessen Leiterelemente (62, 66) elektrisch mit einer Strom- oder Spannungsquelle verbunden sind.

9. Behandlungssystem (90) nach Anspruch 8, dessen Strom- oder Spannungsquelle für eine bedarfsweise pulsierende Beaufschlagung der Elektroden mit Strom oder Spannung ausgelegt ist.

10. Behandlungssystem (90) nach Anspruch 8 oder 9, das für eine Arbeitsspannung an den Elektroden von bis zu 30 V ausgelegt ist.

11. Behandlungssystem (90) nach einem der Ansprüche 8 bis 10, an dessen Behandlungselement (30, 30', 30") ein Zuleitungssystem für eine Reinigungsflüssigkeit angeschlossen ist.

12. Behandlungssystem (90) nach Anspruch 11, bei dem als Reinigungsflüssigkeit Wasser, versetzt mit mindestens einer Säure und/oder mindestens einem Salz, vor gesehen ist.

## Claims

1. A treatment element (30, 30', 30", 30"') for use with a dental implant part (2) inserted into a patient's jaw bone, having a base body (40) provided for mechanical connection with the dental implant part (2) and including at least one media channel (56) for a cleaning liquid,
wherein
the base body (40) carries a first conductor element (62), which forms an electrical current path and can be connected to the dental implant part (2), and a second conductor element (66), which is electrically insulated from the first conductor element (62) and which forms an electrical current path to a contact (64) arranged on the end side and whose contact (64) can be positioned in a spatial area (36) in the vicinity of the dental implant part (2),
**characterized in that**
the second conductor element (66) is formed by one or several of the media channels (56).

2. The treatment element (30, 30', 30") of claim 1, whose base body (40) includes a connection system adapted to the dental implant part (2), for mechanical connection therewith.

3. The treatment element (30, 30', 30") of any of claims 1 or 2, whose second conductor element (66) is designed as an electrode or channel element (80) which is mounted on the treatment element (30, 30', 30") in a displaceable manner in a longitudinal direction which is substantially parallel to the central axis of the base body (40).

4. The treatment element (30, 30', 30") of any of claims 1 to 3, wherein the second conductor element (66) is fixed on the base body (40) in such a way that its contact (64) arranged on the end side can be positioned, viewed in lateral direction, at a distance of maximally 10 mm, preferably of maximally 5 mm, from the central longitudinal axis of the dental implant part (2).

5. The treatment element (30, 30', 30") of any of claims 1 to 4, whose base body (40) is electrically insulated on its contact surface (42) against the dental implant part (2).

6. The treatment element (30, 30', 30") of any of claims 1 to 5, whose connection system comprises a connecting screw (46) for insertion into the screw channel of the post part (2) of a two-part or multi-part dental implant system (1).

7. The treatment element (30, 30', 30") of claim 6, wherein the connecting screw (46) is connected to the first conductor element (62) in an electrically conductive manner.

8. A treatment system (90) for a dental implant part (2) with a treatment element (30, 30', 30") of any of claims 1 to 7, whose conductor elements (62, 66) are electrically connected to a current or voltage source.

9. The treatment system (90) of claim 8, whose current or voltage source is designed for applying a pulsating current or voltage to the electrodes, as needed.

10. The treatment system (90) of claim 8 or 9, which is designed for an operating voltage on the electrodes of up to 30 V.

11. The treatment system (90) of any of claims 8 to 10, on whose treatment element (30, 30', 30") a supply system for a cleaning liquid is connected.

12. The treatment system (90) of claim 11, wherein water, mixed with at least one acid and/or at least one salt, is provided as cleaning liquid.

## Revendications

1. Elément de traitement (30, 30', 30", 30"') destiné à être utilisé avec une partie d'implant dentaire (2) mise en place dans l'os de mâchoire d'un patient, ayant un corps de base (40) prévu pour être relié mécaniquement à la partie d'implant dentaire (2) et comprenant au moins un canal de média (56) pour un liquide de nettoyage,
dans lequel
le corps de base (40) porte un premier élément conducteur (62) qui forme un chemin de courant électrique et peut être relié à la partie d'implant dentaire (2), et un deuxième élément conducteur (66) que est isolé électriquement du premier élément conducteur (62) et qui forme un chemin de courant électrique vers un contact (64) disposé sur le côté frontal et dont le contact (64) peut être positionné dans une zone spatiale (36) à proximité de la partie d'implant dentaire (2),
**caractérisé en ce que**
le deuxième élément conducteur (66) est formé par un ou plusieurs des canaux de média (56).

2. Elément de traitement (30, 30', 30") selon la revendication 1, dont le corps de base (40) comprend un système de connexion adapté à la partie d'implant dentaire (2), pour être relié mécaniquement à cette dernière.

3. Elément de traitement (30, 30', 30") selon l'une quelconque des revendications 1 ou 2, dont le deuxième élément conducteur (66) est conçu comme une électrode ou un élément de canal (80) qui est monté sur l'élément de traitement (30, 30', 30") de façon déplaçable en un sens longitudinal qui est essentiellement parallèle à l'axe central du corps de base (40).

4. Elément de traitement (30, 30', 30") selon l'une quelconque des revendications 1 à 3, dans lequel le deuxième élément conducteur (66) est fixé sur le corps de base (40) de façon que son contact (64) disposé sur le côté frontal peut être positionné, vu dans le sens latéral, à une distance d'au maximum 10 mm, de préférence d'au maximum 5 mm, de l'axe central longitudinal de la partie d'implant dentaire (2).

5. Elément de traitement (30, 30', 30") selon l'une quelconque des revendications 1 à 4, dont le corps de base (40) est isolé électriquement sur sa surface de contact (42) contre la partie d'implant dentaire (2).

6. Elément de traitement (30, 30', 30") selon l'une quelconque des revendications 1 à 5, dont le système de connexion comprend une vis de connexion (46) destinée à être introduite dans le canal fileté de la partie pilier (2) d'un système d'implant dentaire (1) en deux ou plusieurs pièces.

7. Elément de traitement (30, 30', 30") selon la revendication 6, dans lequel la vis de connexion (46) est reliée au premier élément conducteur (62) de façon conductrice électriquement.

8. Système de traitement (90) pour une partie d'implant dentaire (2) avec une élément de traitement (30, 30', 30") selon l'une quelconque des revendications 1 à 7, dont les éléments conducteurs (62, 66) sont reliés électriquement à une source de courant ou de tension.

9. Système de traitement (90) selon la revendication 8, dont la source de courant ou de tension est conçue pour appliquer un courant ou une tension pulsatoire aux électrodes, selon besoin.

10. Système de traitement (90) selon la revendication 8 ou 9, qui est conçu pour une tension de régime de jusqu'à 30 V aux électrodes.

11. Système de traitement (90) selon l'une quelconque des revendications 8 à 10, à l'élément de traitement (30, 30', 30") duquel un système d'alimentation pour un liquide de nettoyage est relié.

12. Système de traitement (90) selon la revendication 11, dans lequel de l'eau mélangé avec au moins un acide et/ou au moins un sel, est prévu comme liquide de nettoyage.
